# EUROPÄISCHE PATENTSCHRIFT

(11) **EP 3 506 958 B1**
(45) Veröffentlichungstag und Bekanntmachung des Hinweises auf die Patenterteilung: **23.06.2021**
(21) Anmeldenummer: 16763488.0
(22) Anmeldetag: 02.09.2016
(51) Int. Cl.: A61M 1/06

(54) **BRUSTHAUBE**
BREAST CUP
TÉTERELLE

(43) Veröffentlichungstag der Anmeldung: 10.07.2019
(60) Teilanmeldung: 19201833.1
(73) Patentinhaber: Medela Holding AG, 6340 Baar (CH)
(72) Erfinder: LARSSON, Michael, 6300 Zug (CH); PFENNIGER, Erich, 6030 Ebikon (CH); RIGERT, Mario, 6033 Buchrain (CH); HÖNER, Sebastian, 8003 Zürich (CH); VISCHER, Peter, 6403 Küssnacht am Rigi (CH); HARTMANN, Peter, Gooseberry Hill, WA 6076 (AU)
(74) Vertreter: Clerc, Natalia
(86) Internationale Anmeldenummer: PCT/EP2016/070742
(87) Internationale Veröffentlichungsnummer: WO 2018/041365

(56) Entgegenhaltungen:
- EP-A1- 1 593 402
- WO-A1-2014/094187
- WO-A1-2016/145173
- CN-U- 201 469 750
- US-A- 4 607 596
- US-A1- 2005 059 928
- US-A1- 2011 301 533
- US-A1- 2014 031 744
- US-B2- 7 988 661

## Beschreibung

### TECHNISCHES GEBIET

Die vorliegende Erfindung betrifft eine Brusthaube zum Abpumpen menschlicher Muttermilch.

### STAND DER TECHNIK

Manuell und motorisch betriebene Brustpumpeneinheiten zum Abpumpen menschlicher Muttermilch sind bekannt. Sie weisen eine Brusthaube oder zwei Brusthauben zur dichtenden Auflage auf die Mutterbrust auf. Diese mindestens eine Brusthaube ist direkt oder über eine Saugleitung mit einer manuell oder motorisch angetriebenen Brustpumpe verbunden. Mittels der Brustpumpe wird ein sich zyklisch änderndes Vakuum erzeugt, welches an die Brusthaube übermittelt wird, um Milch aus der Mutterbrust zu pumpen.

Die Brustpumpeneinheiten sollen ein Abpumpen der Muttermilch ermöglichen, welches dem natürlichen Saugen eines Babys möglichst nahe kommt. Dabei werden spezielle Pumpsequenzen mit sich änderndem Unterdruck und Pumpfrequenz verwendet. Zudem werden Brusthauben in sehr unterschiedlichen Ausführungsformen angeboten, welche dank weichen Einsätzen, sogenannten Linern, ein bequemes Anliegen an die Mutterbrust gewährleisten sollen. Ferner sind Brusthauben bekannt, welche durch Massage die Mutterbrust stimulieren sollen.

Die klassischen Brusthauben weisen einen Trichter zur dichtenden Auflage und zur Aufnahme der Mutterbrust auf. Der Trichter endet in einen rohrförmigen Stutzen, welcher über einen Adapter einerseits direkt oder über eine Saugleitung mit der Brustpumpe und andererseits mit einem Milchsammelbehälter verbindbar ist. Bei Gebrauch ragt die Brustwarze in diesen Stutzen hinein und wird beim Anlegen des zyklisch sich ändernden Unterdrucks in den Stutzen hineingezogen. Der Stutzen sollte dabei so gross sein, dass er die Bewegung der Brustwarze nicht behindert.

Damit das angelegte Vakuum möglichst optimal genutzt werden kann, wird versucht, das zu evakuierende Volumen so klein wie möglich zu halten. Es wird somit versucht, das Totvolumen zu minimieren.

US 4 607 596 offenbart eine Vorrichtung, deren Grundprinzip sich in einem Melkapparat für Vieh wie auch zum Abpumpen von menschlicher Muttermilch verwenden lassen soll. Die zugehörige Brusthaube weist einen steifen Grundkörper mit einem flexiblen Einsatz auf. Es sind zwei mit einem pulsierenden Unterdruck beaufschlagbare Kammern vorhanden, wobei der Unterdruck von derselben Brustpumpe generiert wird. Die erste Kammer ist durch den Innenraum gebildet, in welcher die Mutterbrust aufgenommen wird. Die zweite Kammer befindet sich zwischen dem flexiblen Einsatz und dem Grundkörper.

Bei US 7 988 661 B2 weist die Brusthaube ebenfalls zwei Kammern auf, wobei diese mit unterschiedlichem und voneinander unabhängigem Druck beaufschlagbar sind. Es lassen sich insbesondere ein Unterdruck sowie ein Überdruck anlegen. Diese Publikation zeigt eine Vielzahl unterschiedlicher Brusthauben. Die Figuren 16A und 16 zeigen eine Brusthaube mit drei Kammern, welche ein Saugen eines Babys simulieren sollen. Die Brustwarze wird dabei beim Anlegen des Unterdrucks in die Länge gezogen. Die drei Kammern lassen sich unabhängig voneinander mit Druck beaufschlagen, so dass auch eine Rotationsbewegung um die Längsachse der Brusthaube simuliert werden kann. In der Ausführungsform gemäss Figur 17 weist der flexible Einsatz in einem hohlzylindrischen Bereich nach innen gerichtete Rippen auf, welche die Brustwarze und das angrenzende Gewebe der Mutterbrust massieren und stimulieren. In den Ausführungsformen gemäss den Figuren 18 und 19 bilden die Kammern Einbuchtungen zur Brustwarze hin, um diese so zu massieren.

WO 2014/094186 A2 beschreibt eine Brusthaubeneinheit mit einem flexiblen Einsatz zur Aufnahme der Mutterbrust und der Brustwarze sowie mit einer separaten Medientrennmembran zum Schutz der Vakuumquelle. Die Bewegung der Medientrennmembran ist derart, dass sie die Brustwarze nicht kontaktiert und somit die Bewegung der Brustwarze nicht behindert.

WO 2014/063261 A1 offenbart eine Brusthaube mit einem flexiblen Einsatz, welcher gleichzeitig als Medientrennmembran dient. Dieser flexible Einsatz ist verdreht in einer hohlzylinderförmigen Aufnahme gehalten und nimmt die Brustwarze auf. Bei Anlegen eines Unterdrucks zwischen Aufnahme und Einsatz vergrössert der Einsatz seinen Milchdurchlass.

WO 2011/037841 A2 zeigt eine Brusthaube, welche im Übergang zwischen dem Trichter und dem rohrförmigen Stutzen aufblasbare Kissen aufweist.

US 9 248 223 B2 beschreibt eine Brusthaube mit einem weichen Einsatz, welcher mittels Unterdruck einen peristaltischen Druck auf die Brustwarze ausübt, um Milch abzupumpen. WO 2016/007561 A1 zeigt einen Brusthaubeneinsatz, welcher im Bereich der Brustwarze mit Rillen versehen ist, um die Oberfläche zu vergrössern.

US 2016/0058928 A1 offenbart eine Brusthaube, welche dem Mund eines Babys entsprechen soll. Die Brustwarze wird in einem flexiblen Brusthaubenteil aufgenommen, welcher asymmetrisch kollabieren kann, um die Mundbewegung des Babys zu imitieren. Der Unterdruck wird über den Milchsammelbehälter an die Brusthaube geleitet WO2016/145173 gehört zum Stand der Technik gemäß Artikel 54(3) EPÜ und offenbart eine Brusthaube mit Sensorik. US2005/059928 offenbart eine Brusthaube mit Sensoren.

Obwohl diese neueren Brusthauben teilweise gute Lösungsansätze zeigen, um dem natürlichen Saugen nahe zu kommen, ist eine optimale Imitation der Natur und somit die optimale Form und Druckbeaufschlagung einer Brusthaube noch nicht gefunden worden.

### DARSTELLUNG DER ERFINDUNG

Es ist deshalb eine Aufgabe der Erfindung, eine alternative Brusthaube sowie eine Brustpumpeneinheit zu schaffen, welche eine maximierte Abpumpleistung sowie eine minimierte Abpumpdauer pro Pumpsitzung ermöglichen.

Diese Aufgabe löst eine Brusthaube mit den Merkmalen des Patentanspruchs 1 sowie eine Brustpumpeneinheit mit den Merkmalen des Patentanspruchs 15 bzw. 17.

In einem nicht-erfindungsgemäßen Verfahren zum Betreiben einer Brustpumpeneinheit zum Abpumpen von menschlicher Muttermilch weist die Brustpumpeneinheit eine Vakuumpumpe zur Erzeugung von Drücken und mindestens eine Brusthaube auf zur dichtenden Auflage an eine abzupumpende Mutterbrust. Die Brusthaube weist eine innere Kammer zur Aufnahme einer Brustwarze der Mutterbrust sowie mindestens eine äussere Kammer auf, welche die Brustwarze mindestens teilweise umgibt. Erfindungsgemäss wird die innere Kammer mit einem ersten Druck der Vakuumpumpe versehen und die mindestens eine äussere Kammer mit mindestens einem zweiten Druck der Vakuumpumpe. Für den ersten Druck wird ein annähernd zeitlich konstanter Druck und für den mindestens einen zweiten Druck ein pulsierender Druck verwendet. Alternativ wird für den ersten Druck ein pulsierender Druck und für den mindestens einen zweiten Druck ein annähernd zeitlich konstanter Druck verwendet.

Der annähernd zeitlich konstante Druck ist ein Druck, welcher entweder über den gesamten Zeitraum des Pumpvorgangs konstant bleibt oder welcher sich im Vergleich zum zweiten pulsierenden Druck zeitlich um ein Vielfaches langsamer ändert.

Unter "pulsierenden Druck" wird ein sich verändernder Druck verstanden, welcher sich vorzugsweise zyklisch verändert. Vorzugsweise verändert sich der Druck gleichmässig, z.B. sinusförmig. Er kann sich jedoch auch innerhalb eines Zyklus ungleichmässig verändern und/oder es können regelmässige oder unregelmässige Pausen zwischen den Zyklen vorhanden sein.

Im Stand der Technik wird im Hohlraum der Brusthaube, in welchem die Brustwarze aufgenommen wird, ein pulsierender, d.h. ein sich verändernder Druck angelegt, welcher die Saugaktion eines Säuglings simulieren soll. Durch den angelegten Unterdruck wird die Brustwarze während des Abpumpens gedehnt und in die Länge gezogen. Im Gegensatz dazu wird die Brustwarze bei Anwendung des nicht-erfindungsgemäßen Verfahrens, nachfolgend erstes Verfahren genannt, nicht oder kaum gedehnt. Hingegen werden durch das Anlegen des äusseren, sich verändernden Drucks die in der Brustwarze verlaufenden natürlichen Milchkanäle offen gehalten, regelmässig geöffnet und/oder noch zusätzlich radial erweitert, so dass die Milch ungehemmt ausfliessen kann.

Der im Hohlraum der Brusthaube, d.h. direkt an die Brustwarze angelegte konstante Unterdruck beeinflusst die Form der Brustwarze kaum und dient mehrheitlich dem Halten der Position der Brusthaube und dem Abführen der abgepumpten Muttermilch.

Vorzugsweise liegen die Werte des ersten Drucks und des mindestens einen zweiten Drucks in einem Bereich, in welchem die Brustwarze der Mutterbrust in ihrer Länge im Wesentlichen unverändert bleibt.

Dieses Verfahren erlaubt somit eine maximierte Abpumpleistung. Da der Durchmesser der Milchkanäle der Brustwarze maximiert wird, ist zudem eine minimierte Abpumpdauer pro Pumpsitzung möglich.

In einer Variante des nicht-erfindungsgemäßen Verfahrens weist die Brusthaube ein flexibles Innenteil auf, einen sogenannten Liner. Dieses flexible Innenteil unterteilt die Brusthaube in die innere Kammer und in die mindestens eine äussere Kammer. Das flexible Innenteil wird von innen mit dem ersten Druck und von aussen mit dem mindestens einen zweiten Druck beaufschlagt.

Vorzugsweise wird zur Positionierung der Brusthaube auf der Mutterbrust in einem ersten Schritt der erste Druck in der ersten Kammer angelegt, damit das flexible Innenteil nach innen gezogen wird zur Kontaktierung der Brustwarze, und in einem weiteren Schritt wird der mindestens eine zweite Druck angelegt. Das Totvolumen wird dadurch minimiert. Die Brustwarze wird massiert und der äussere, sich verändernde Druck lässt sich optimal an die Brustwarze anlegen.

In einer alternativen Variante wird zur Positionierung der Brusthaube auf der Mutterbrust in einem ersten Schritt in der mindestens einen zweiten Kammer mindestens ein dritter Druck angelegt wird, wobei der dritte Druck zeitlich konstant ist, wobei das flexible Innenteil durch diesen dritten Druck nach aussen gezogen wird zur Bildung eines Innenraums zwecks Aufnahme der Brustwarze, wobei in einem weiteren Schritt der erste und der mindestens eine zweite Druck angelegt werden, um Milch abzupumpen. Dadurch wird das Gewebe der Brustwarze beim Platzieren innerhalb der Brusthaube optimal geschont und der flexible Innenteil kann anschliessend über den gesamten Umfang eng an der Brustwarze anliegen.

Vorzugsweise wird für den ersten Druck ein Unterdruck und für den mindestens einen zweiten Druck ein Unterdruck und/oder ein Überdruck verwendet. Indem der zweite Druck ein sich verändernder Unterdruck ist, welcher zeitweise in einen Überdruck wechselt, ist eine grosse Bandbreite an Möglichkeiten zur Aktivierung und Massage der Brustwarze gegeben.

Vorzugsweise werden der erste Druck und der mindestens eine zweite Druck unabhängig voneinander verwendet. Auch dies erhöht die Bandbreite der oben genannten Möglichkeiten.

Vorzugsweise werden der erste Druck und der mindestens eine zweite Druck nach Massgabe einer Steuerungseinheit in Abhängigkeit voneinander angelegt.

Es kann genau eine zweite Kammer vorhanden sein. Diese Art der Brusthauben lässt sich einfach und kostengünstig herstellen. In einer anderen Ausführungsform sind mindestens zwei zweite Kammern vorhanden, welche unabhängig voneinander mit je einem zweiten Druck versehen werden. Vorzugsweise wird dabei das Verhältnis der mindestens zwei zweiten Drücke relativ zueinander zeitlich verändert. Dies ermöglicht ein möglichst naturnahes Massieren der Brustwarze während des Abpumpens, d.h. eine Beanspruchung der Brustwarze ähnlich zur Situation im Mund des Säuglings.

In einem weiteren nicht-erfindungsgemäßen Verfahren zum Betreiben einer Brustpumpeneinheit zum Abpumpen von menschlicher Muttermilch weist die Brustpumpeneinheit eine Vakuumpumpe zur Erzeugung von Drücken und mindestens eine Brusthaube auf zur dichtenden Auflage an eine abzupumpende Mutterbrust. Die Brusthaube weist ein flexibles Innenteil mit einer inneren Kammer zur Aufnahme einer Brustwarze der Mutterbrust und mindestens eine äussere Kammer auf, welche die Brustwarze mindestens teilweise umgibt. Die innere Kammer wird mit einem ersten Druck der Vakuumpumpe versehen und die mindestens eine äussere Kammer wird mit mindestens einem zweiten Druck der Vakuumpumpe versehen. Das flexible Innenteil wird derart druckbeaufschlagt, dass es in einer ersten Position ringförmig an der Brustwarze anliegt und dass es in einer zweiten Position die Brustwarze in radialer Richtung freigibt.

Dieses nachfolgend zweites Verfahren genannte Verfahren erlaubt ebenfalls ein Abpumpen der Muttermilch, ohne dass die Brustwarze in die Länge gezogen wird und ohne dass sich die Durchmesser der natürlichen Milchkanäle verkleinern. Je nach Art der Druckbeaufschlagung lässt sich zudem eine Vergrösserung der lichten Weite der natürlichen Milchkanäle erreichen.

Im Stand der Technik ist die Brustwarze in der Brusthaube kontaktfrei aufgenommen. Die Brusthaube liegt üblicherweise ausschliesslich am angrenzenden Brustgewebe an. Diese Brusthauben massieren das Gewebe der Brust. Im zweiten Verfahren wird hingegen die Brustwarze kontaktiert, vorzugsweise eng umschlossen, und je nach Variante des zweiten Verfahrens massiert. Vorzugsweise wird ausschliesslich die Brustwarze oder maximal noch ein Teil oder die ganze Areola kontaktiert.

In einer Variante des nicht-erfindungsgemäßen zweiten Verfahrens ist der erste Druck pulsierend und der mindestens eine zweite Druck konstant. In einer anderen Variante ist dies umgekehrt.

Die nicht-erfindungsgemäßen Verfahren, insbesondere das oben beschriebene erste Verfahren, lassen sich beispielweise mit nachfolgend beschriebenen Brustpumpeneinheiten und Brusthauben optimal einsetzen. Die nachfolgend beschriebenen Brustpumpeneinheiten und Brusthauben lassen sich jedoch auch mit anderen Verfahren betreiben.

Eine erste erfindungsgemässe Brustpumpeneinheit zum Abpumpen von menschlicher Muttermilch weist eine Vakuumpumpe zur Erzeugung von Drücken und mindestens eine Brusthaube gemäß Anspruch 1 zur dichtenden Auflage an eine abzupumpende Mutterbrust auf. Die Brusthaube weist eine innere Kammer zur Aufnahme einer Brustwarze der Mutterbrust sowie mindestens eine äussere Kammer auf, welche die Brustwarze mindestens teilweise umgibt. Die innere Kammer ist zur Aufnahme eines ersten Drucks der Vakuumpumpe ausgebildet und die mindestens eine äussere Kammer ist zur Aufnahme mindestens eines zweiten Drucks der Vakuumpumpe ausgebildet. Der erste Druck ist dabei ein annähernd zeitlich konstanter Druck und der mindestens eine zweite Druck ist ein pulsierender Druck.

Die Brusthaube dieser ersten Brustpumpeneinheit weist vorzugsweise ein flexibles Innenteil auf, welches die Brusthaube in die innere Kammer und die mindestens eine äussere Kammer unterteilt. Das flexible Innenteil ist von innen mit dem ersten Druck und von aussen mit dem mindestens einen zweiten Druck beaufschlagbar. Derartige flexible Innenteile werden oft Liner genannt. Der Liner kann lösbar in einem steifen oder halbsteifen Brusthaubenkörper gehalten sein oder er kann gemeinsam mit ihm gefertigt und nicht zerstörungsfrei von ihm lösbar sein.

Diese erste Brustpumpeneinheit weist mindestens einen Sensor zur Bestimmung der Position der Brustwarze während des Abpumpvorgangs auf. Dadurch lässt sich feststellen, ob und gegebenenfalls wieviel die Brustwarze durch die angelegten Drücke gedehnt oder gestaucht wird. Vorzugsweise ist die Steuerung ausgebildet, den ersten Druck und/oder den mindestens einen zweiten Druck nach Massgabe dieser bestimmten Position der Brustwarze zu variieren. Dadurch lässt sich die Brustpumpe individuell an die Bedürfnisse der Mutter anpassen. Es ist dadurch für jede Mutter möglich, abzupumpen, ohne dass ihre Brustwarze zu stark gedehnt oder die Milchkanäle der Brustwarze zu stark verkleinert werden. Der mindestens eine Sensor kann zusätzlich oder alternativ zur Bestimmung der Stelle bezüglich der Längsachse der Brusthaube verwendet werden, an welcher die innere Kammer kollabiert bzw. das flexible Innenteil die Kammer verschliesst.

Nachfolgend sind verschiedene Ausführungsformen von Brusthauben erwähnt, welche sich insbesondere in den oben genannten Verfahren und in den in diesem Text beschriebenen Brustpumpeneinheiten einsetzen lassen. Diese Brusthauben weisen jeweils einen Auflagebereich zur dichtenden Auflage auf die menschliche Mutterbrust und eine innere Kammer zur Aufnahme einer Brustwarze der Mutterbrust auf.

Eine erste dieser erfindungsgemässen Brusthauben weist mindestens eine äussere Kammer auf, welche die Brustwarze mindestens teilweise umgibt. Die innere Kammer ist zur Aufnahme eines ersten Drucks der Vakuumpumpe ausgebildet und die mindestens eine äussere Kammer ist zur Aufnahme mindestens eines zweiten Drucks der Vakuumpumpe ausgebildet. Der erste Druck ist ein annähernd zeitlich konstanter Druck und der mindestens eine zweite Druck ist ein pulsierender Druck.

Da die Brusthaube lediglich die Brustwarze umfassen muss, kann sie relativ klein ausgebildet werden. Sie lässt sich auch versteckt und diskret in einer Freihandlösung (hands-free) unter der Kleidung benützen. Zudem ist das Totvolumen minimiert, so dass auch das die zwei Drücke erzeugende Brustpumpenaggregat entsprechend klein ausgebildet sein kann. Dies minimiert die Kosten und optimiert die Leistung.

Diese erste Brusthaube weist vorzugsweise ein flexibles Innenteil auf, welches die Brusthaube in die innere Kammer und die mindestens eine äussere Kammer unterteilt. Das flexible Innenteil ist von innen mit dem ersten Druck und von aussen mit dem mindestens einen zweiten Druck beaufschlagbar. Vorzugsweise ist das flexible Innenteil eine flexible Einlage, welche fest oder lösbar mit einem Brusthaubenkörper verbunden ist. Das flexible Innenteil vereinfacht die Herstellung der Brusthaube. Zudem ermöglicht es ein dichtendes Anliegen an die Brustwarze und eine angenehme und wirkungsvolle Massage sowie eine optimale Stimulation der Brustwarze.

Bei einer zweiten dieser erfindungsgemässen Brusthauben ist die innere Kammer über den gesamten Aufnahmebereich konisch ausgebildet. Die konische Ausbildung verhindert, dass die Brustwarze durch den angelegten Unterdruck zu stark in die Länge gezogen wird und somit die Durchmesser der natürlichen Milchkanäle verkleinert werden. Zudem ermöglicht die konische Form ein optimales anliegendes Umfassen der Brustwarze über die gesamte Länge der Brustwarze.

Die innere Kammer dieser zweiten Brusthaube weist eine innere Wand auf, welche zusätzlich zur konischen Form oder alternativ zu dieser konischen Form mit Rückhaltemitteln zum Rückhalten der Brustwarze während des Pumpvorgangs ausgestattet ist. Diese Rückhaltemittel verhindern, dass die Brustwarze beim Abpumpen in die Länge gezogen wird.

Eine dritte dieser erfindungsgemässen Brusthauben ist mit mindestens einem Sensor versehen zur Bestimmung der Position der Brustwarze während des Abpumpvorgangs. Wie bereits oben erwähnt, lassen sich die Drücke nach Massgabe dieses Messsignals so einstellen, dass die Längenveränderung der Brustwarze optimiert wird. Insbesondere ist die Veränderung minimiert.

Die innere Kammer einer vierten dieser erfindungsgemässen Brusthauben weist eine Längsachse auf. Die innere Kammer kollabiert nach Massgabe eines angelegten Drucks. Die Brusthaube ist mit mindestens einem Sensor versehen zur Bestimmung der Lage, an welcher die innere Kammer kollabiert. Dieses Kollabieren hält ebenfalls die Brustwarze zurück und verhindert somit eine unerwünschte Dehnung derselben. Dank des Sensors lässt sich feststellen, ob die innere Kammer an der gewünschten Stelle kollabiert. Falls nicht, lässt sich der angelegte Druck bzw. lassen sich die angelegten Drücke verändern und/oder die Platzierung der Brusthaube auf der Brustwarze kann korrigiert werden.

Der Auflagebereich einer fünften der erfindungsgemässen Brusthauben endet brustseitig in einem umlaufenden, weichen und dichtenden Kissen. Das Kissen weist vorzugsweise einen umlaufenden aufblasbaren Hohlraum auf. Dieses Kissen ermöglicht ein druckfreies und doch dichtendes Anliegen an die Brustwarze oder die Areola. Dies ist bei empfindlichen oder bereits entzündeten Brüsten für die Mutter angenehm. Zudem erzeugt die Brusthaube auch bei zu starkem Anpressen der Brusthaube durch die Mutter keine Knickstelle auf der Brust. Der Milchfluss wird nicht beeinträchtigt oder beeinflusst.

Eine sechste der erfindungsgemässen Brusthauben weist brustseitig eine umlaufende Aufnahmetasche zum Auffangen von Muttermilchtropfen beim Entfernen der Brusthaube auf. Beim Entfernen der Brusthaube von der Brust können somit keine Tropfen verloren gehen. Es lässt sich die gesamte Muttermilch bis auf den letzten Tropfen verwenden. Dies ist insbesondere bei Frühgeburten wichtig, wenn die Mutter in der ersten Zeit noch kaum selber Milch produzieren kann.

Vorzugsweise weist diese sechste Brusthaube ein flexibles Innenteil auf, welches den Auflagebereich zur dichtenden Auflage auf die menschliche Mutterbrust und eine innere Kammer zur Aufnahme einer Brustwarze der Mutterbrust bildet, wobei die umlaufende Tasche in dem flexiblen Innenteil ausgebildet ist. Vorzugsweise ist der flexible Innenteil nach aussen stülpbar, so dass die aufgefangenen Milchtropfen sich einfacher aus der Brusthaube entnehmen lassen.

Eine siebte der erfindungsgemässen Brusthauben weist einen äusseren Brusthaubenkörper und ein flexibles Innenteil auf, wobei das flexible Innenteil den Auflagebereich zur dichtenden Auflage auf die menschliche Mutterbrust bildet. Das flexible Innenteil unterteilt die Brusthaube in die innere Kammer zur Aufnahme einer Brustwarze der Mutterbrust und in mindestens eine äussere Kammer, welche die Brustwarze mindestens teilweise umgibt. Die innere Kammer ist zur Aufnahme eines ersten Drucks der Vakuumpumpe ausgebildet und die mindestens eine äussere Kammer ist zur Aufnahme mindestens eines zweiten Drucks der Vakuumpumpe ausgebildet. Das flexible Innenteil ist einstückig ausgebildet. Die Brusthaube weist eine weitere Kammer in Form eines Hohlraums auf, welche von der mindestens einen äusseren Kammer unterteilt ist, indem eine feste oder lösbare Verbindung zwischen dem flexiblen Innenteil und dem äusseren Brusthaubenkörper eine umlaufende Trennwand bildet. Der Hohlraum ist im Auflagebereich der Brusthaube angeordnet. Dadurch lässt sich ein aufblasbares Kissen schaffen, um die Brusthaube optimal auf der Brustwarze zu platzieren. Dasselbe Unterteilungsprinzip lässt sich auch benutzen, um mehr als eine äussere Kammer zu schaffen, welche mit unterschiedlichen Drücken beaufschlagt werden können, um so die Brustwarze an unterschiedlichen Stellen unterschiedlich zu massieren und zu stimulieren.

Eine achte der erfindungsgemässen Brusthauben definiert eine Längsachse. Die innere Kammer ist durch einen äusseren Bereich begrenzt, welcher asymmetrisch ausgebildet ist. Mindestens ein Teilbereich des äusseren Bereichs weist eine äussere Kammer auf, deren Innenseite mit einem Druck beaufschlagbar ist. Vorzugsweise ist ein Teilbereich des äusseren Bereichs in seiner Steifigkeit und/oder Härte veränderbar, beispielsweise durch ein steifes Einstellelement. Diese Brusthaube imitiert den Mund eines Säuglings mit Gaumen und Zunge.

Eine zweite erfindungsgemässe Brusthaubeneinheit einer Brustpumpeneinheit zum Abpumpen von menschlicher Muttermilch weist eine Vakuumpumpe zur Erzeugung von Drücken auf. Die Brusthaubeneinheit umfasst eine Brusthaube mit einem Innenraum zur Aufnahme einer Brustwarze und einen flexiblen Milchsammelbehälter. Der Innenraum weist eine erste Öffnung zur Aufnahme der Brustwarze und als ausschliessliche weitere Öffnung eine Verbindungsöffnung zum Milchsammelbehälter auf, wobei die Brusthaube über diese Öffnung luftdicht mit dem Milchsammelbehälter verbunden ist. Es sind Mittel vorhanden, welche den Innenraum zyklisch vergrössern zwecks Erzeugung eines Unterdrucks im Innenraum zum Abpumpen der Muttermilch. Diese zweite Brusthaubeneinheit kann eine Weiterbildung der oben beschriebenen ersten Brusthaubeneinheit sein. Diese Brusthaubeneinheit minimiert den Kontakt der Milch mit der Umgebungsluft, so dass eine Kontamination der Milch weitgehend vermieden werden kann. Dies ist insbesondere im Neonatologiebereich wichtig.

Die Mittel dieser zweiten Brusthaubeneinheit sind vorzugsweise Federzungen und die Federzungen betätigende Leinen. Eine derartige Brusthaubeneinheit lässt sich kostengünstig und einfach herstellen. Auch ihre Benützung ist relativ einfach. Sie lässt sich wiederum im Neonatologiebereich optimal einsetzen.

Eine neunte der erfindungsgemässen Brusthauben weist einen Lüfter auf, welcher Luft in Richtung Mutterbrust bläst. Diese an die Brust geblasene Luft imitiert das Atmen des Babys und fördert somit die Milchproduktion der Mutter.

Die Erfindung ist eine Brusthaube gemäß Anspruch 1. Weitere Ausführungsformen sind in den abhängigen Ansprüchen angegeben.

### KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

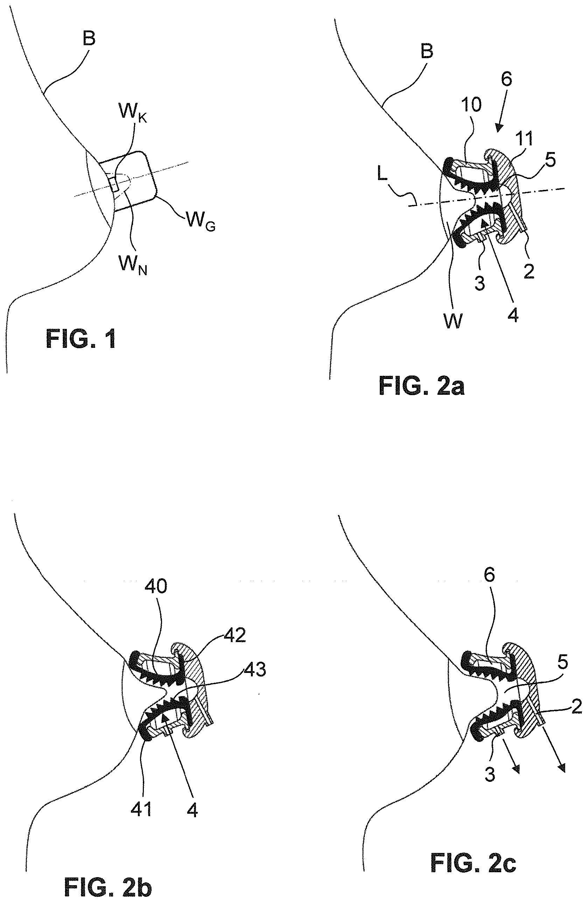

- Figur 1: eine schematische Darstellung einer menschlichen Mutterbrust mit möglichen Formen der Brustwarze;
- Figur 2a: eine schematische Darstellung einer Brusthaube in einer ersten Ausführungsform bevor ein Druck angelegt ist;
- Figur 2b: die Brusthaube gemäss Figur 2a mit in einer inneren Kammer angelegtem Druck, z.B. Vakuum;
- Figur 2c: die Brusthaube gemäss Figur 2a mit in der inneren und in einer äusseren Kammer angelegten Drücken;

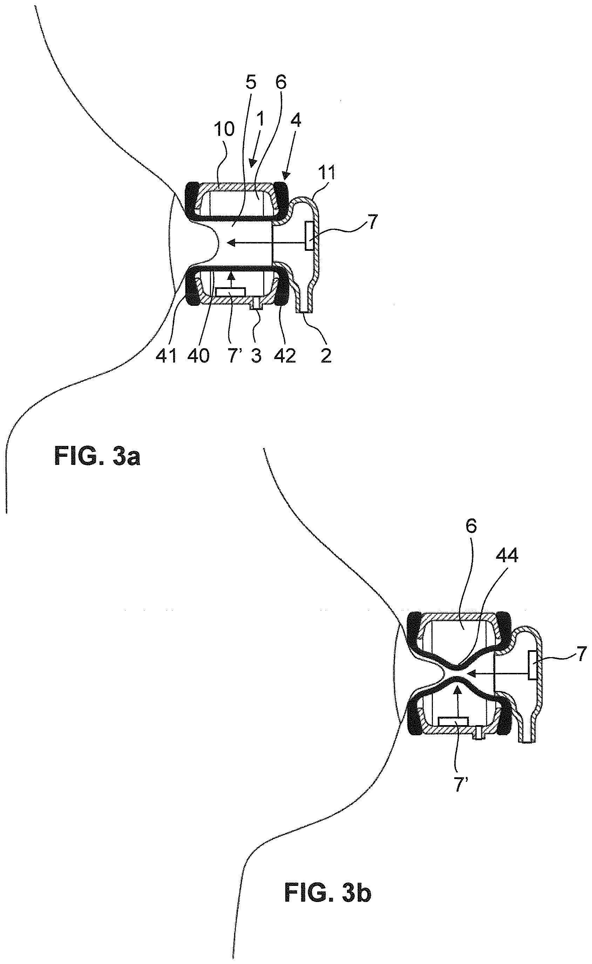

- Figur 3a: eine schematische Darstellung einer erfindungsgemässen Brusthaube in einer zweiten Ausführungsform mit Sensoren, in einer Ausgangsposition;
- Figur 3b: die Brusthaube gemäss Figur 3a mit in einer inneren Kammer angelegtem Druck;

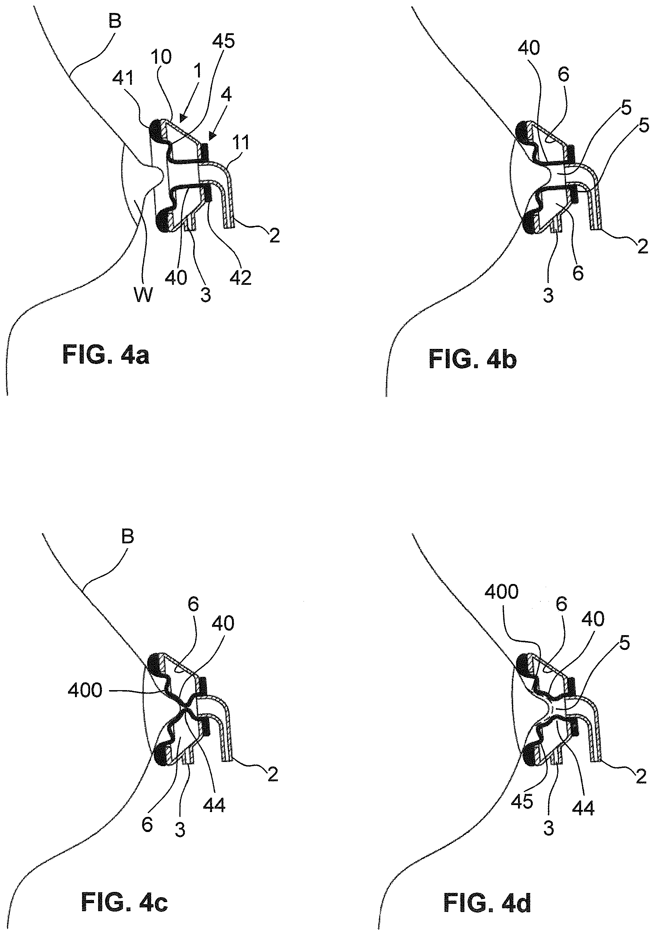

- Figur 4a: eine schematische Darstellung einer Brusthaube in einer vierten Ausführungsform vor Anlegen an die Mutterbrust;
- Figur 4b: die Brusthaube gemäss Figur 4a bei Anlage auf der Mutterbrust;
- Figur 4c: die Brusthaube gemäss Figur 4a mit in einer inneren Kammer angelegtem Druck;
- Figur 4d: die Brusthaube gemäss Figur 4a mit in der inneren und in einer äusseren Kammer angelegten Drücken;

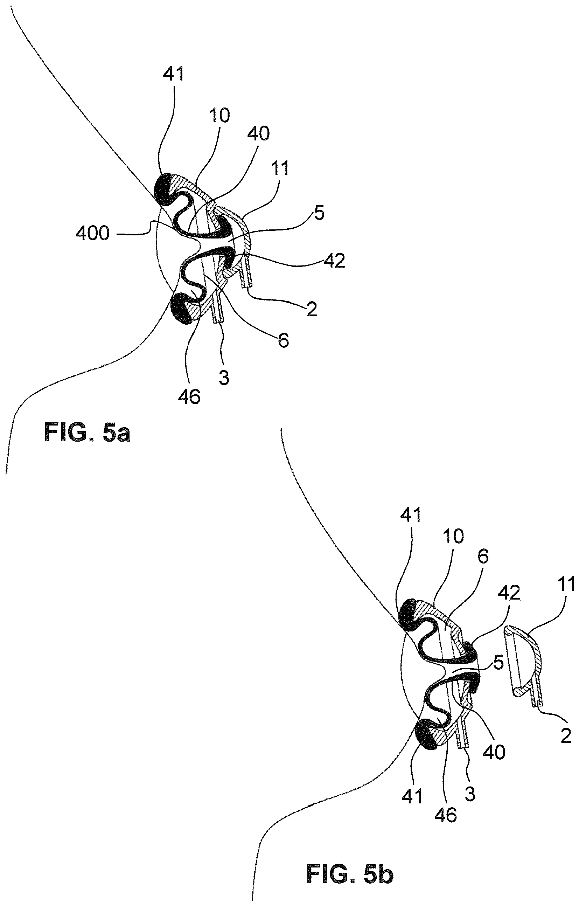

- Figur 5a: eine schematische Darstellung einer Brusthaube in einer vierten Ausführungsform bei Anlage auf der Mutterbrust;
- Figur 5b: die Brusthaube gemäss Figur 5a mit entferntem Deckel;

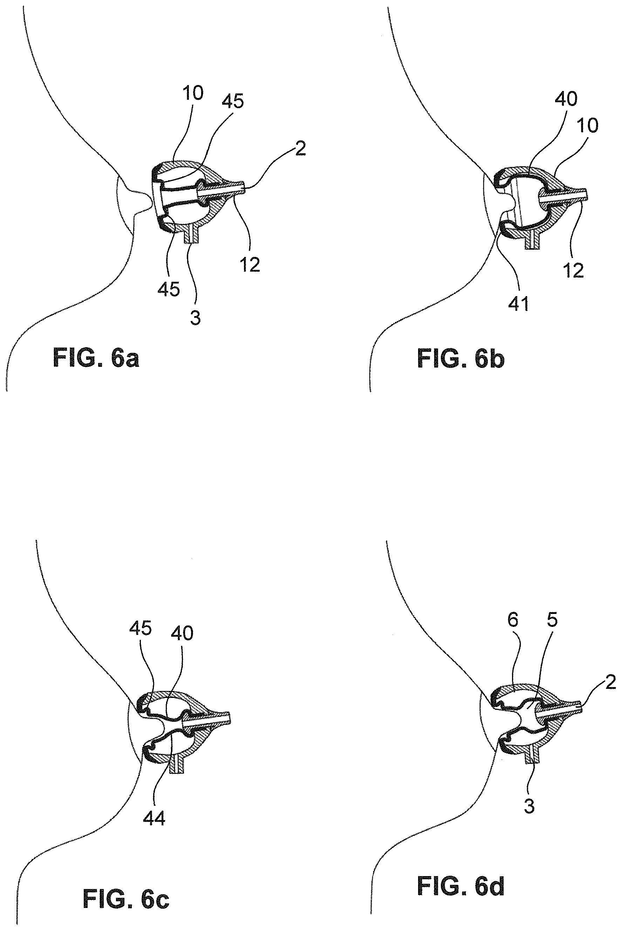

- Figur 6a: eine schematische Darstellung einer Brusthaube in einer fünften Ausführungsform vor Anlegen an die Mutterbrust;
- Figur 6b: die Brusthaube gemäss Figur 6a im vollständig geöffneten Zustand nach Anlegen an die Mutterbrust;
- Figur 6c: die Brusthaube gemäss Figur 6a im geschlossenen Zustand nach Anlegen an die Mutterbrust;
- Figur 6d: die Brusthaube gemäss Figur 6a während des Abpumpens von Muttermilch;

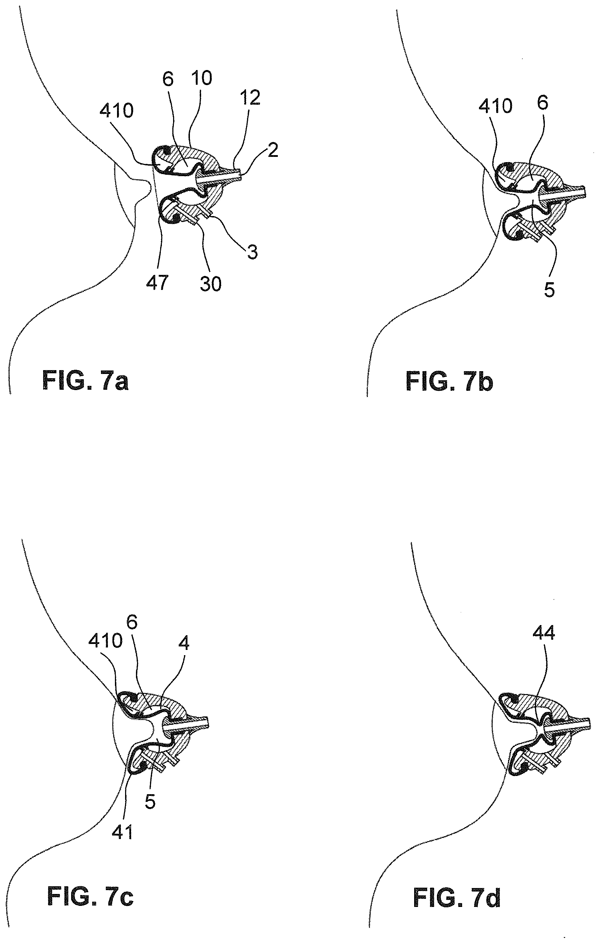

- Figur 7a: eine schematische Darstellung einer Brusthaube in einer sechsten Ausführungsform vor Anlegen an die Mutterbrust;
- Figur 7b: die Brusthaube gemäss Figur 7a beim Anlegen an die Mutterbrust;
- Figur 7c: die Brusthaube gemäss Figur 7a nach Anlegen an die Mutterbrust;
- Figur 7d: die Brusthaube gemäss Figur 7a während des Abpumpens von Muttermilch;

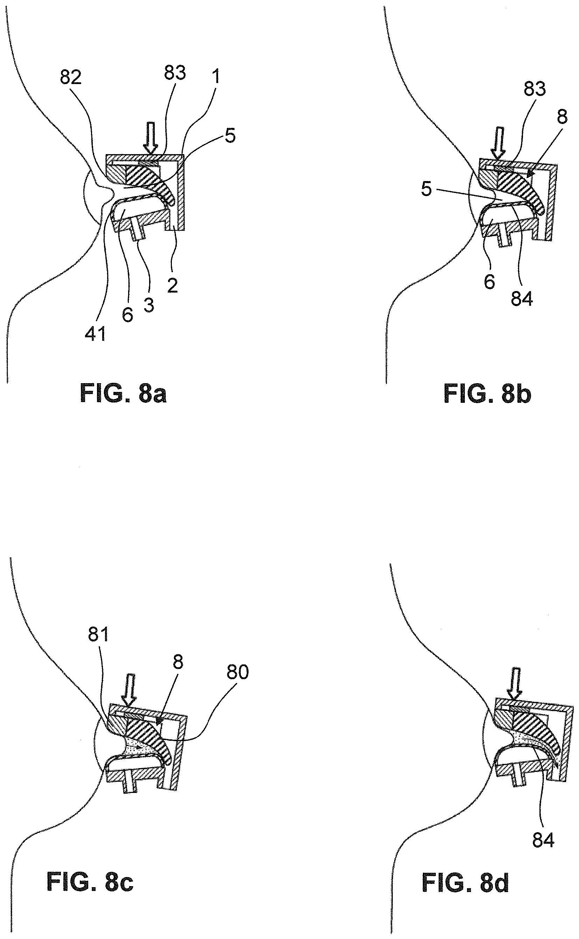

- Figur 8a: eine schematische Darstellung einer Brusthaube in einer siebten Ausführungsform vor Anlegen an die Mutterbrust;
- Figur 8b: die Brusthaube gemäss Figur 8a beim Anlegen an die Mutterbrust;
- Figur 8c: die Brusthaube gemäss Figur 8a während des Abpumpens von Muttermilch in einer ersten Situation;
- Figur 8d: die Brusthaube gemäss Figur 8a während des Abpumpens von Muttermilch in einer zweiten Situation;

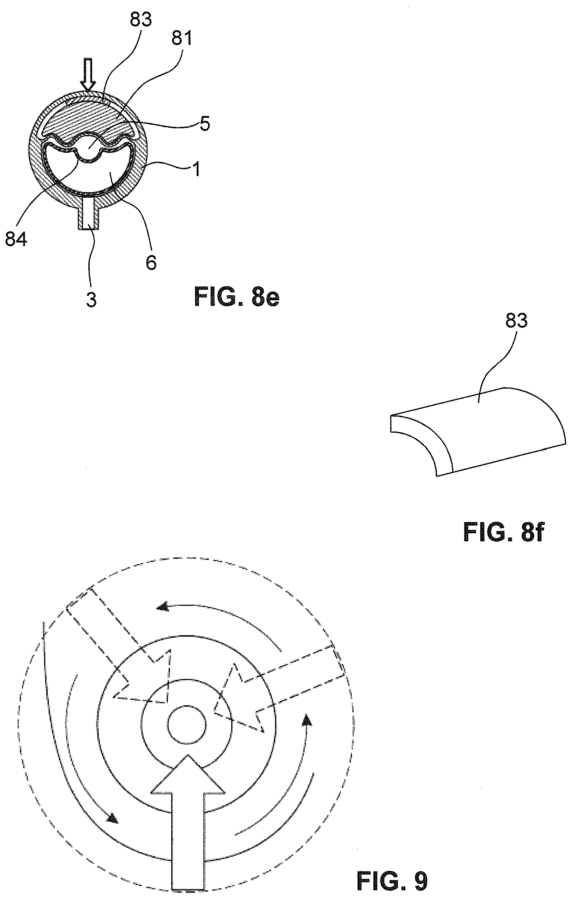

- Figur 8e: einen Querschnitt durch die Brusthaube gemäss Figur 8a;
- Figur 8f: eine perspektivische schematische Darstellung eines Einstellelements der Brusthaube gemäss Figur 8a;
- Figur 9: eine schematische Darstellung der beim Abpumpen auf eine Brustwarze wirkenden Kräfte von vorne zur Brust hin gesehen;

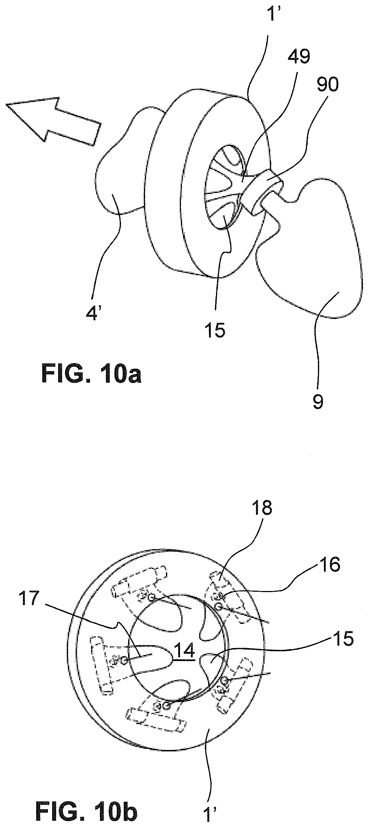

- Figur 10a: eine perspektivische schematische Darstellung einer Brustpumpeneinheit mit Brusthaube und Milchsammelbehälter in einer achten Ausführungsform;
- Figur 10b: eine perspektivische schematische Darstellung eines Teils der Brusthaube gemäss Figur 10a;

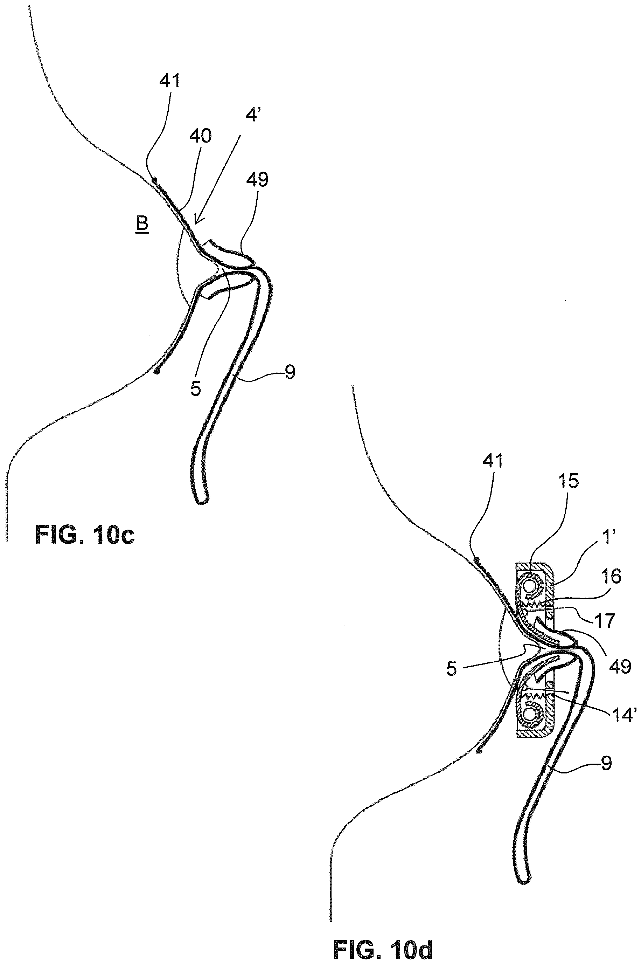

- Figur 10c: einen schematischen Querschnitt durch die Brusthaube mit Milchsammelbehälter gemäss Figur 10a vor Gebrauch;
- Figur 10d: einen schematischen Querschnitt durch die Brustpumpeneinheit gemäss Figur 10a vor Gebrauch;

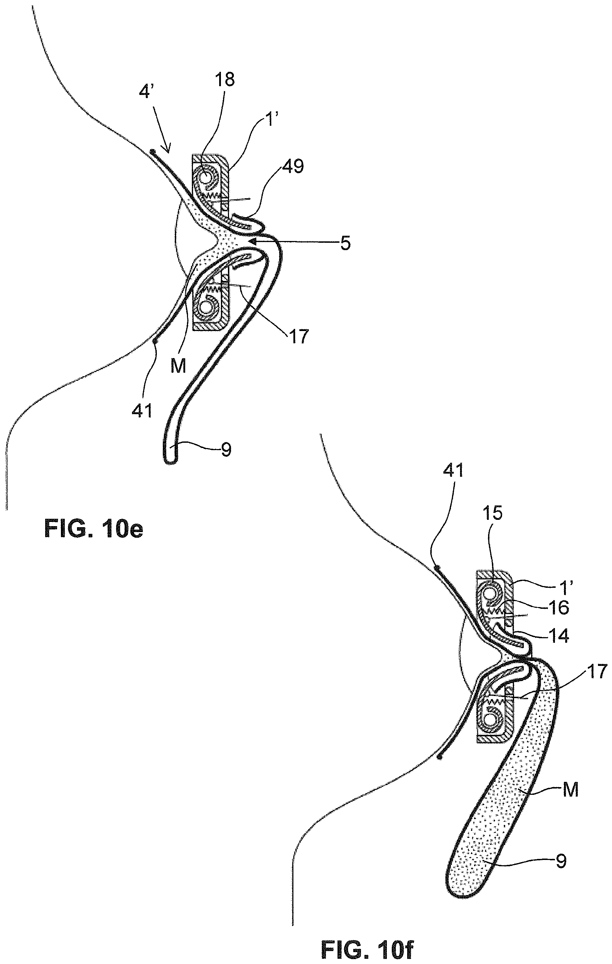

- Figur 10e: die Brustpumpeneinheit gemäss Figur 10d beim Abpumpen in einer ersten Position;
- Figur 10f: die Brustpumpeneinheit gemäss Figur 10d beim Abpumpen in einer zweiten Position;

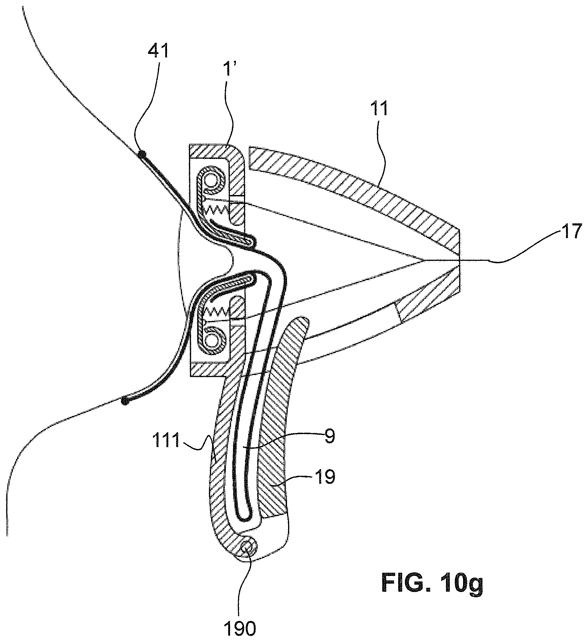

- Figur 10g: eine Variante der Brustpumpeneinheit gemäss Figur 10d;

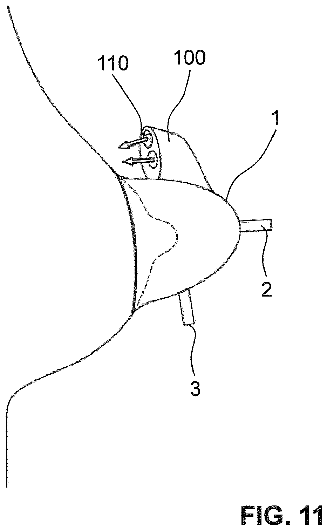

- Figur 11: eine perspektivische schematische Darstellung einer Brusthaube mit einem Milchsammelbehälter in einer neunten Ausführungsform;

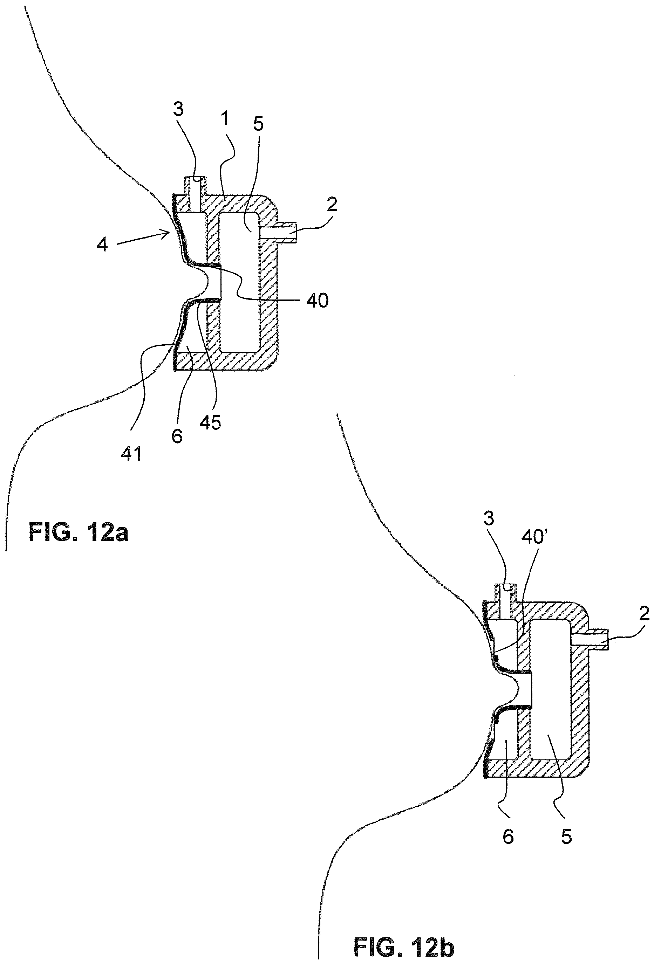

- Figur 12a: eine schematische Darstellung einer Brusthaube mit einem Milchsammelbehälter in einer zehnten Ausführungsform und
- Figur 12b: eine Variante der Brusthaube gemäss Figur 12a und

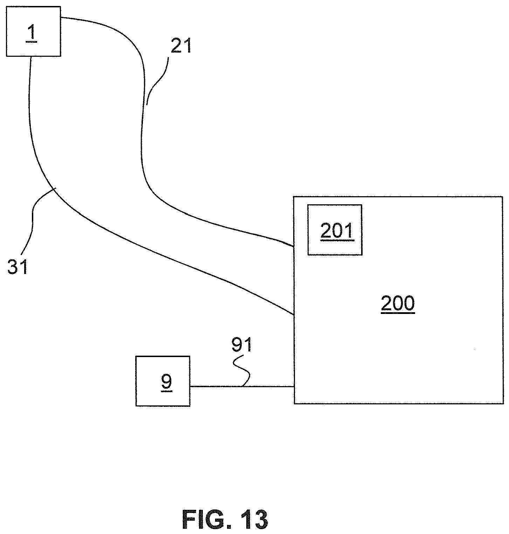

- Figur 13: eine schematische Darstellung einer Brustpumpeneinheit.

Gleiche oder ähnliche Elemente sind mit gleichen Bezugszeichen versehen.

### BESCHREIBUNG BEVORZUGTER AUSFÜHRUNGSFORMEN

Figur 1 zeigt eine schematische Darstellung einer menschlichen Mutterbrust B mit einer Brustwarze W_{N} in Durchschnittsgrösse, einer kleinen Brustwarze W_{K} und einer grossen Brustwarze W_{G}. Der Durchmesser der Brustwarzen verschiedener Mütter weist eine Bandbreite von circa 10 mm bis circa 24 mm auf mit einem Durchschnittswert von ca. 16 mm. Die Länge der Brustwarze ohne äussere Beeinflussung variiert von Mutter zu Mutter von circa 3 mm bis circa 20 mm mit einem Durchschnittswert von circa 7 mm.

Die Brusthauben im Stand der Technik haben die Brustwarzen üblicherweise nicht kontaktiert, so dass die Varianz der Brustwarzen unterschiedlicher Mütter unbeachtet bleiben konnte. Die erfindungsgemässen Brusthauben liegen jedoch bevorzugt an den Brustwarzen an und sollen diese durch enges Anliegen und radiales Loslassen stimulieren, um Milch zu extrahieren. Vorzugsweise liegen die Brusthauben sogar ausschliesslich an der Brustwarze oder nur noch zusätzlich auf der Areola, aber nicht auf dem umliegenden Brustgewebe der Mutterbrust an. Die nachfolgend beschriebenen Brusthauben lassen sich vorzugsweise für die gesamte oben erwähnte Bandbreite von möglichen Brustwarzen einsetzen, entweder indem sie selber entsprechend in unterschiedlichen Grössen angeboten werden oder noch bevorzugter, indem sie sich durch ihre Form und, falls vorhanden, ihrem flexiblen Innenteil, an Form und Grösse der jeweiligen Brustwarze anpassen.

In den Figuren 2a bis 2c ist eine erste Ausführungsform einer erfindungsgemässen Brusthaube dargestellt. Sie weist einen steifen oder halbsteifen Brusthaubenkörper 1 auf, welcher vorzugsweise aus Kunststoff gefertigt ist. In diesem Beispiel ist der Brusthaubenkörper 1 zweiteilig gestaltet. Er weist eine Basis 10 sowie einen Deckel 11 auf.

Der Deckel 11 weist einen ersten Vakuumanschluss 2 zur Verbindung mit einer Vakuumpumpe auf. Die Vakuumpumpe weist mindestens ein Vakuumaggregat zur Erzeugung eines Unterdrucks auf. Die Vakuumpumpe ist in Figur 13 dargestellt und wird später in diesem Text beschrieben.

Die Basis 10 ist in diesem Beispiel im Wesentlichen kegelstumpfförmig ausgebildet. Sie kann auch eine andere Form aufweisen, beispielsweise kann sie hohlzylinderförmig ausgebildet sein. Sie weist in diesem Beispiel einen brustseitigen und einen pumpenseitigen Befestigungsflansch auf. An der Basis 10 ist ein zweiter Vakuumanschluss 3 vorhanden, welcher eine Verbindung zu vorzugsweise demselben Pumpaggregat oder zu einem anderen Pumpaggregat der Vakuumpumpe ermöglicht.

Die Basis 10 und der Deckel 11 umschliessen einen Hohlraum, welcher von einem flexiblen Innenteil 4, auch Liner genannt, in eine innere Kammer 5 und eine äussere Kammer 6 unterteilt ist.

Die innere Kammer 5 weist eine brustseitige Öffnung auf, durch welche die Brustwarze W bei Gebrauch in die Brusthaube eingeführt wird. Pumpenseitig endet der erste Vakuumanschluss 2 in der inneren Kammer 5 und verbindet diese somit mit der Vakuumpumpe. Die innere Kammer 5 weist vorzugsweise nur diese zwei Öffnungen auf. In anderen Ausführungsformen weist die innere Kammer 5 noch einen Milchanschluss auf.

Die äussere Kammer 6 ist vorzugsweise bis auf den zweiten Vakuumanschluss 3 vollständig geschlossen ausgebildet. Die Wände der äusseren Kammer 6 werden vorzugsweise durch den steifen oder halbsteifen Brusthaubenkörper 1 und das flexible Einsatzelement 4 gebildet.

Das flexible Innenteil 4 ist über die Basis 10 gestülpt und wird durch sie in dieser Position gehalten. Es kann auch angespritzt sein. Vorzugsweise besteht es aus einem weichen Kunststoff, vorzugsweise aus Silikon. Ist es ein loses Teil, so ist es vorzugsweise mittels des Deckels 11 klemmend gehalten.

Das flexible Innenteil 4 weist einen Grundkörper 40, einen umlaufenden Auflagebereich 41 und einen umlaufenden Befestigungsflansch 42 auf. Der Befestigungsflansch 42 ist zwischen Deckel 11 und Basis 10 eingeklemmt. Mit dem Auflagebereich 41 liegt die Brusthaube im bestimmungsgemässen Gebrauch auf der Brustwarze W und/oder der sie umgebenden Areola dichtend auf oder an. Der Auflagebereich 41 ist in diesem Beispiel die Unterseite des verdickten Flansches, welcher über die Basis 10 gestülpt ist. Dadurch ist er zwar brustseitig weich, auf seiner Rückseite jedoch durch die Basis 10 stabilisiert, so dass die Mutter von Hand oder durch einen hands-free Büstenhalter einen genügenden Anpressdruck zur dichtenden Anlage ausüben kann. Der verdickte Bereich ist beispielsweise als umlaufendes hohles oder massives Kissen ausgebildet.

Der Grundkörper 40 kann sich zwischen diesen zwei Flanschen relativ zur Längsmittelachse L der Brusthaube bewegen, wie durch die Zusammenschau der Figuren 2a, ab und 2c ersichtlich ist.

Der Grundkörper 40 kann glattwandig ausgeführt sein. In dieser Ausführungsform weist es Rückhalteelemente 43 für die Brustwarze W auf. Die Brustwarze W kann sich auch bei angelegtem Unterdruck in der inneren Kammer 5 nicht zu stark dehnen, da sie durch die Rückhalteelemente 43 in ihrer Ausdehnung begrenzt wird.

Vorzugsweise sind die Rückhalteelemente 43 durch umlaufende Rippen gebildet, welche sich über mindestens einen Teil der Länge des Grundkörpers erstrecken. Die Rippen sind vorzugsweise zur Brustseite hin gewandt. Sie können aber auch radial nach innen zur Längsmittelachse L der Brusthaube hin ragen. Vorzugsweise verjüngen sich die Rippen zu ihrem freien Ende hin. Sie können aber auch eine andere Form aufweisen, z.B. abgerundete freie Enden aufweisen. Die Rippen sind vorzugsweise relativ weich ausgebildet, um die Brustwarze W nicht zu reizen oder gar zu verletzen. Sie sind vorzugsweise jedoch steif genug, um während des Abpumpens der Milch eine übermässige Verlängerung der Brustwarze W zu verhindern. Anstelle von Rippen lassen sich auch andere Arten von Rückhalteelementen 43 einsetzen, beispielsweise durch geeignete Wahl eines Materials einer an sich glatten inneren Wand des Grundkörpers 40, d.h. durch Wahl eines Materials mit einem genügend grossen Reibungskoeffizienten.

In der Situation gemäss Figur 2a ist die Brusthaube auf der Brustwarze W aufgesetzt und umgibt diese dichtend. Es ist noch kein Vakuum angelegt. Der Grundkörper 40 des flexiblen Innenteils 4 liegt beabstandet zur Brustwarze W oder kontaktiert diese leicht, ohne einen wesentlichen Druck auf sie auszuüben. Die Brustwarze W hat ihre von äusseren Kräften unbeeinflusste natürliche Form.

In der Situation gemäss Figur 2b ist über den ersten Vakuumanschluss 2 mittels der Vakuumpumpe ein zeitlich annähernd konstanter Unterdruck angelegt. Er kann über den gesamten nachfolgenden Abpumpvorgang konstant bleiben bzw. nach Massgabe der Mutter oder einer Steuerung der Vakuumpumpe angepasst, aber über einen nachfolgenden Zeitraum bis zur nächsten Anpassung wiederum konstant sein. Er kann sich jedoch auch zyklisch ändern, wobei die Zyklusdauer sehr lang ist, z.B. eine oder mehrere Minuten beträgt. Alternativ oder zusätzlich kann sich auch der Mittelwert des Zyklus verändern.

Wie in Figur 2b gut erkennbar ist, wird der Grundkörper 40 des flexiblen Innenteils 4 aufgrund des in der inneren Kammer 5 herrschenden Unterdrucks nach innen zur Längsmittelachse L hin gezogen. Die Brustwarze W wird kontaktiert und fest umschlossen. Die Rückhaltemittel 43 verhindern jedoch, dass die Brustwarze W gleichzeitig übermässig in die Länge gezogen wird. Vorzugsweise beträgt die mögliche Verlängerung der Brustwarze W nur wenige Prozent, vorzugsweise weniger als 20%.

In der Situation gemäss Figur 2c ist der konstante Unterdruck in der inneren Kammer 5 weiterhin beibehalten. Über den zweiten Vakuumanschluss 3 ist in der äusseren Kammer 6 gleichzeitig ein pulsierender Unterdruck angelegt, welcher zusätzlich einen Überdruckanteil aufweisen kann. Vorzugsweise bewegt sich der angelegte Unterdruck jedoch zwischen einem maximalen Unterdruck und Atmosphärendruck oder weist sogar ein stets vorhandenes Basisvakuum auf. Der zweite maximale Unterdruck ist vorzugsweise im Absolutwert grösser als der erste maximale Unterdruck, d.h. die äussere Kammer 6 wird stärker evakuiert als die innere Kammer 5.

Durch das Anlegen des pulsierenden Unterdrucks in der äusseren Kammer 6 wird der Grundkörper 40 des flexiblen Innenteils 4 wieder nach aussen und von der Längsmittelachse L der Brusthaube weg gezogen. Der Grundkörper kann sich wieder entspannen und er wölbt sich dabei wieder nach aussen. Dieser Massageeffekt führt dazu, dass sich die Brustwarze W wieder entspannt und sich die natürlichen Milchkanäle der Brustwarze W erweitern.

Milch fliesst in dieser dritten Situation aus der Brustwarze W in die innere Kammer 5. Je nach Ausführungsform der Brusthaube kann ein weiterer Anschluss vorhanden sein, welcher direkt oder über eine Leitung mit einem Milchsammelbehälter verbunden ist. In dieser Ausführungsform fliesst die Milch durch den ersten Vakuumanschluss 2 zur Brustpumpe und von dort in den Milchsammelbehälter. D.h. die Vakuumleitung für den konstanten Unterdruck dient gleichzeitig als Milchleitung.

Durch Anlegen des konstanten Drucks in der inneren Kammer 5 und des pulsierenden Drucks in der äusseren Kammer 6 lässt sich das erfindungsgemässe Verfahren durchführen, welches die Brustwarze W entspannt und vorzugsweise nach aussen zieht und dadurch die natürlichen Milchkanäle öffnet. Diese Brusthaube lässt sich jedoch auch in anderen Verfahren verwenden, z.B. indem die innere Kammer 5 mit einem sich pulsierenden, d.h. sich zyklisch ändernden Unterdruck beaufschlagt wird und die äussere Kammer 6 je nach Variante mit einem pulsierenden und/oder mit einem konstanten Druck beaufschlagt wird. Dies führt zu einem Massageeffekt. Dasselbe gilt auch für die nachfolgend beschriebenen Brusthauben.

In den Figuren 3a und 3b ist eine zweite Ausführungsform einer erfindungsgemässen Brusthaube dargestellt. Der Grundaufbau der Brusthaube ist derselbe wie im ersten Ausführungsbeispiel und wird hier deshalb nicht mehr im Detail erläutert. Es ist wiederum der steife oder halbsteife Brusthaubenkörper 1 vorhanden, in welchem das flexible Innenteil 4 angeordnet ist. Der erste Vakuumanschluss führt in die innere Kammer 5 und der zweite Vakuumanschluss 3 in die äussere Kammer 6. Die äussere Kammer 6 umläuft die Aussenseite des flexiblen Innenteils 4. Die Innenwandung des Grundkörpers 40 ist in dieser Darstellung glatt ausgebildet. In anderen Varianten ist sie ebenfalls mit Rückhalteelementen 43, beispielsweise mit Rippen, versehen. In der inneren Kammer 5 ist vorzugsweise wiederum ein konstanter Unterdruck angelegt und in der äusseren Kammer 6 ist ein diesen im Betrag übersteigender pulsierender Unterdruck vorhanden. D.h. auch hier lässt sich da erfindungsgemässe Verfahren anwenden, in welchem die Verlängerung der Brustwarze W begrenzt wird, und die Brustwarze W durch den Unterdruck in der äusseren Kammer 6 mittels des flexiblen Innenteils 4 massiert und gegebenenfalls radial gedehnt wird.

Figur 3a zeigt die Situation, in welcher kein Unterdruck oder in beiden Kammern 5, 6 ein Unterdruck angelegt ist. Figur 3b zeigt die Situation, wenn nur in der inneren Kammer 5 ein Unterdruck angelegt ist oder dieser zumindest betragsmässig überwiegt.

In Figur 3b ist erkennbar, wie der Grundkörper 40 nach innen zur Längsmittelachse L der Brusthaube hin gezogen ist, wobei der Grundkörper 40 den Durchgang, gebildet zwischen dem brustseitigen Ende der Brusthaube und dem pumpenseitigen Ende der Brusthaube, teilweise oder ganz verschliesst. In der Figur 3b ist er noch nicht vollständig geschlossen. Der Verschluss 44 findet vorzugsweise unmittelbar vor dem freien Ende der Brustwarze W statt, so dass diese durch den Verschluss 44 an einer weiteren Ausdehnung in Längsrichtung gehindert wird. Der Verschluss 44 bildet somit ein Rückhaltemittel für die Brustwarze W.

Auch in dieser Ausführungsform kann die Innenseite des Grundkörpers 40 entweder mit einer glatten Oberfläche oder mit zusätzlichen Rückhaltemitteln versehen sein.

Alle Ausführungsformen der Erfindung weisen mindestens einen, vorzugsweise zwei Sensoren 7, 7' auf. Der erste Sensor 7 ist in der Verlängerung der Brustwarze W, hier im Deckel 11, angeordnet und misst entlang der Längsmittelachse L der Brusthaube. Er detektiert die Position der Spitze der Brustwarze W sowie des Verschlusses 44. Der zweite Sensor 7' ist radial zum Grundkörper 40 des flexiblen Innenteils 4 angeordnet und detektiert die radiale Bewegung des Grundkörpers 40. Beide Sensoren 7, 7' sind vorzugsweise optische Sensoren. Anstelle eines einzigen zweiten Sensors 7' lassen sich auch mehrere über den Umfang der Brusthaube verteilt angeordnete Sensoren 7' verwenden.

Mit Hilfe dieser zwei Sensoren 7, 7' lässt sich die Position des Verschlusses 40 und die Veränderung der Brustwarze W bestimmen. Diese Sensoren 7, 7' sind vorzugsweise mit einer optischen und/oder akustischen Anzeige und/oder mit einer Steuerung der Brustpumpe verbunden. Nach Massgabe dieser Messsignale lassen sich die Pumpparameter, wie beispielsweise die Pumpfrequenz und/oder das Vakuumlevel verändern, so dass der Verschluss 44 an einer optimalen Stelle für die betreffende Grösse der Brustwarze W zu liegen kommt und somit die Brustwarze W in ihrer Dehnung in Längsrichtung optimal begrenzen kann.

In den Figuren 4a bis 4d ist eine dritte Ausführungsform der erfindungsgemässen Brusthaube dargestellt. Der Brusthaubenkörper 1 ist hier einstückig ausgebildet und weist wiederum die zwei Anschlüsse 2 und 3 und die innere Kammer 5 und die äussere Kammer 6 auf. Das flexible Innenteil 4 ist über beide Stirnflächen des Brusthaubenkörpers 1 gestülpt und so gehalten. Der Grundkörper 40 des flexiblen Innenteils ist im Wesentlichen hohlzylinderförmig ausgebildet, wobei er zu seinem brustseitigen Ende hin in einen nach aussen gerichteten, umlaufenden und in sich geschlossenen Bogen 400 übergeht. Dieser Bogen 400 kann dieselbe Wandstärke aufweisen wie der zylinderförmige Teil des Grundkörpers 40. Er kann jedoch auch verdickt ausgeführt sein. Der Grundkörper 40 kann im Mehrkomponenten- insbesondere im Zweikomponenten-Spritzgrussverfahren hergestellt sein.

Der umlaufende brustseitige Flansch des flexiblen Innenteils 4 ist nach aussen gerichtet und bildet wiederum den umlaufenden, in sich geschlossenen Auflagebereich 41 zur Auflage auf die Brustwarze W oder die angrenzende Areola. Der Auflagebereich 41 ist vorzugsweise verdickt ausgeführt. Vorzugsweise ist er relativ weich, ähnlich einem umlaufenden Kissen.

Die innere Kammer 5 wird wiederum mit einem konstanten Vakuum beaufschlagt, die äussere Kammer 6 mit einem pulsierenden, insbesondere mit einem sich zyklisch ändernden Vakuum versehen.

In Figur 4a ist die Brusthaube im Grundzustand dargestellt, bevor sie an die Brust gelegt wird. In Figur 4b ist die Brusthaube auf der Brustwarze W platziert und umschliesst diese. Dabei ist das freie Ende der Brustwarze W in den hohlzylinderförmigen Teil des Grundkörpers 40 aufgenommen. Dieser Teil kann auch eine andere Form aufweisen. Z.B. kann er kegelstumpfförmig sein.

In Figur 4c ist die innere Kammer 5 mit dem konstanten Druck beaufschlagt. Der Grundkörper 40 des flexiblen Innenteils wird zur Längsmittelachse L der Brusthaube gezogen, der Bogen 400 verändert seine Form und der Grundkörper 40 bildet wiederum einen Verschluss 44. Dadurch wird die Brustwarze W wiederum in ihrer Längsdehnung behindert, ohne dass sie übermässigen externen Kräften ausgesetzt wird. Wird nun der Innenraum der äusseren Kammer 6 mit dem pulsierenden Vakuum versehen, so bewegt sich der Grundkörper 40 mindestens teilweise wieder radial nach aussen und gibt die Brustwarze W mindestens teilweise wieder frei. Der Bogen 400 verändert dabei seine Form, liegt jedoch vorzugsweise während des gesamten Abpumpvorgangs an der Brustwarze W an und umgibt diese. Vorzugsweise ist der Bogen 400 so weich und flexibel ausgebildet, dass er keinerlei Druckstellen auf der Brustwarze W erzeugt. Dadurch wird der Milchfluss nicht behindert.

Die Bewegung des Bogens 400 auf der Brustwarze W und/oder der Areola führt zu einer Massage und Stimulation der Brustwarze W und somit zu einem erhöhten Milchausstoss. Das ständige Anliegen des Bogens 400 lässt sich beispielsweise sicherstellen, indem der Betrag des angelegten konstanten Vakuums während des gesamten Zyklus höher liegt als der Betrag des pulsierenden Vakuums.

Die Ausführungsform gemäss den Figuren 5a und 5b ist insbesondere für Mütter mit sehr geringer Milchproduktion, insbesondere von Müttern mit Frühgeburten, optimal einsetzbar. Auch hier werden die bereits oben erläuterten Teile nicht mehr im Detail beschrieben. Die angelegten Drücke sind vorzugsweise wie oben beschrieben. Zusätzlich zu dem vorzugsweise stets an der Brustwarze W anliegendem Bogen 400 ist eine Tasche 46 zur Aufnahme von einzelnen Milchtropfen vorhanden. Die Tasche 46 ist vorzugsweise die Brusthaube umlaufend ausgebildet, so dass sie keinen Einfluss auf die Drehposition der Brusthaube auf der Brustwarze W hat. Abgepumpte Milch, welche nicht durch den Milchkanal, oder je nach Ausführungsform durch den ersten Vakuumanschluss 2, abgesaugt wird, wird in dieser Tasche 46 aufgefangen. Wenn die Brusthaube nach dem Abpumpen von der Brustwarze W entfernt wird, ist diese zusätzliche Milch in der Tasche 46 gehalten und kann ebenfalls noch gesammelt und verwendet werden. Auf diese Weise geht kein Tropfen der kostbaren Muttermilch verloren.

Die Figuren 6a bis 6d zeigen eine weitere Ausführungsform der erfindungsgemässen Brusthaube, welche vorzugsweise mit einem konstanten inneren Druck und einem pulsierenden äusseren Druck betrieben wird.

In dieser Ausführungsform ist das flexible Innenteil 4 an seinem pumpenseitigen Ende über einen Anschlussstutzen 12 gestülpt, welcher den ersten Vakuumanschluss 2 bildet. Der Grundkörper 40 geht auch hier brustseitig in einen Bogen über, welcher einen Kontaktierungsbereich 45 bildet. Dieser Kontaktierungsbereich 45 kontaktiert die Brustwarze W vorzugsweise während des gesamten Abpumpvorgangs, wobei der Kontaktierungsbereich 45 vorzugsweise wie im vorherigen Beispiel über den gesamten Umfang der Brustwarze W an dieser anliegt.

In Figur 6a ist die Brusthaube im Grundzustand dargestellt. Der innere Durchmesser des Grundkörpers 40 ist vorzugsweise gleich oder kleiner als der Durchmesser einer kleinsten oder in Frage kommenden Brustwarze W.

In Figur 6b ist die Brusthaube auf die Brustwarze W aufgelegt, wobei ein konstanter Unterdruck in der äusseren Kammer 6, nicht aber in der inneren Kammer 5 angelegt ist. Dadurch hat sich der Grundkörper 40 des flexiblen Innenteils 4 radial nach aussen bewegt. Die innere Kammer 5, welche der Aufnahme der Brustwarze W dient, hat maximales Volumen erreicht. Die Brusthaube lässt sich dadurch einfach über die Brustwarze W stülpen. Dies ist insbesondere bei empfindlichen oder entzündeten Brustwarzen W vorteilhaft.

Anschliessend wird, wie in Figur 6c dargestellt ist, ein Unterdruck in der inneren Kammer 5 erzeugt und vorzugsweise der Absolutwert des Unterdrucks in der äusseren Kammer 6 vermindert, auf Atmosphärendruck gesetzt oder sogar auf einen Überdruck angehoben. Dadurch wird der Grundkörper 40 zur Längsmittelachse L der Brusthaube und zur Brustwarze W hingezogen. Der Auflagebereich 45 umschliesst die Brustwarze W und liegt über den gesamten Umfang an dieser an. Im Endbereich der Brustwarze W bildet sich wiederum der Verschluss 44 aus. Der effektive Pumpvorgang kann nun beginnen.

Dieser ist in Figur 6d dargestellt. Über den ersten Anschluss 2 ist ein konstantes Vakuum an die innere Kammer 5 angelegt, über den Anschluss 3 ist ein pulsierendes, vorzugsweise betragsmässig höheres Vakuum angelegt.

Während des Pumpvorgangs ändert sich die Form des flexiblen Innenteils 4 von der Form gemäss Figur 6d zur Form gemäss Figur 6c und zurück. Bei Figur 6d wird gepumpt, bei Figur 6c wird massiert und stimuliert.

Die Brusthaube lässt sich nach Beendigung des Pumpvorgangs einfach und schmerzfrei entfernen, wenn wiederum mittels den angelegten Drücken die Situation gemäss Figur 6b herbeigeführt wird.

Diese Ausführungsform hat den weiteren Vorteil, dass das flexible Innenteil 4 keinerlei Rümpfe oder Falten aufweist und dass das flexible Innenteil 4 lediglich durch Druckveränderung beim Auflegen der Brusthaube auf die Mutterbrust in die optimale Passform in Bezug auf die individuelle Brustwarze W gebracht werden kann.

Die Ausführungsform gemäss den Figuren 7a bis 7d unterscheidet sich von der obigen im Wesentlichen dadurch, dass zusätzlich zur äusseren Kammer 6 ein weiterer umlaufender äusserer geschlossener Hohlraum 410 gebildet ist, welcher auch mit Druck beaufschlagbar ist. Entsprechend sind zwei zweite Anschlüsse 3, 30 vorhanden. Dies ist in dieser Ausführungsform dadurch erreicht, dass das flexible Innenteil 4 eine umlaufende Trennwand 47 aufweist, welche den Bereich zwischen dem steifen oder halbsteifen Brusthaubenkörper 1 und dem flexiblen Innenteil 4 in zwei Bereiche unterteilt.

Vorzugsweise ist diese Trennwand 47 so ausgebildet, dass sie über ihren gesamten Umfang mit einem entsprechenden vorstehenden oder versenkt angeordneten Gegenstück der Innenwandung des Brusthaubenkörpers 1 verbunden ist bzw. verbindbar ist.

Die brustferne äussere Kammer 6 wird wiederum zur Bewegung des Grundkörpers 40 des flexiblen Innenteils 4 verwendet, analog zu den bereits oben beschriebenen Beispielen. Der brustnahe aufblasbare Hohlraum 410 bildet ein umlaufendes aufblasbares Kissen für den Auflagebereich 41.

In Figur 7a ist die Brusthaube im Grundzustand dargestellt. Der innere Durchmesser des Brustaufnahmebereichs des flexiblen Innenteils 4 ist vorzugsweise gleich gross oder grösser als der Durchmesser einer Brustwarze W.

In Figur 7b ist sie auf die Brustwarze W aufgelegt. Ein konstanter Überdruck wird über den zweiten Anschluss 30 an den brustnahe Hohlraum 410 angelegt, damit sich der Hohlraum 410 ausdehnt und ein aufgepumptes umlaufendes Kissen bildet, welches an der Brustwarze W und/oder an der Areola anliegt. Die Brustwarze W ist dabei in das flexible Innenteil 4 aufgenommen, wobei sie leicht zusammengepresst wird.

Gemäss Figur 7b wird nun der Überdruck im Kissen, d.h. im Hohlraum 410 reduziert. Vorzugsweise wird Atmosphärendruck oder ein Unterdruck in diesem Hohlraum 410 erzeugt. Die Brustwarze W kann sich dadurch wieder entspannen und in ihrer Länge verkürzen. Sie ist aber nach wie vor im flexiblen Innenteil 4 so gehalten, dass dieser die Brustwarze W über ihren gesamten Umfang kontaktiert.

Anschliessend beginnt der Pumpvorgang, welcher in Figur 7d dargestellt ist. Ein konstanter Unterdruck wird über den ersten Vakuumanschluss 2 in der inneren Kammer 5 erzeugt. Ein pulsierender Unterdruck wird über den zweiten Vakuumanschluss 3 in der pumpennahen und somit brustfernen äusseren Kammer 6 erzeugt. Dies führt wiederum zur Massage und Stimulation der Brustwarze W sowie zum Milchausstoss.

In den Figuren 8a bis 8f ist eine weitere Ausführungsform einer erfindungsgemässen Brusthaube dargestellt. Diese simuliert die Gegebenheiten im Munde eines Säuglings.

Der steife oder halbsteife Brusthaubenkörper ist wiederum mit dem Bezugszeichen 1 versehen. Er weist wiederum einen ersten Vakuumanschluss 2 zur Applikation eines konstanten Drucks, insbesondere eines Vakuums in eine innere Kammer 5 auf. Ein zweiter Vakuumanschluss 3 zur Applikation eines pulsierenden Unterdrucks führt zu einer äusseren Kammer 6. Die Brustwarze W ist wie in den oben beschriebenen Ausführungsbeispielen in der inneren Kammer 5 aufgenommen.

Im Brusthaubenkörper 1 ist ein flexibles Innenteil 8 angeordnet, welches nunmehr nicht mehr einstückig ausgebildet ist, wie in den vorherigen Beispielen. Es weist vielmehr einen oberen Teil 80, 81, 82 auf, welchen den Gaumen des Säuglings imitiert und einen unteren Teil 84, welcher die Zunge des Säuglings imitiert und somit ein Zungenteil bildet. Beide Teile 80, 81, 82, 84 sind vorzugsweise fest mit dem Brusthaubenkörper 1 verbunden, wobei sie relativ zu diesem bewegbar sind, um die Grösse der inneren und äusseren Kammer 5, 6 zu verändern. Der untere Teil 84 begrenzt zusammen mit einem benachbarten Bereich des Brusthaubenkörpers 1 die äussere Kammer 6. Der obere Teil 80, 81, 82 begrenzt zusammen mit einem benachbarten Bereich des Brusthaubenkörpers 1 sowie mit dem unteren Teil 84 die innere Kammer 5.

Der obere Teil weist eine zur Brust hingerichtete stirnseitige Fläche 82 auf, welche als Auflagebereich zur dichtenden Auflage an die Brustwarze W oder die Areola dient. Der untere Teil 84 weist ein entsprechendes Pendant auf, welches als Auflagebereich das Bezugszeichen 41 trägt.

Der obere Bereich 80, 81, 82 kann aus Bereichen mit verschiedener Härte bestehen, indem die Materialien entsprechend gewählt sind. Er kann ein- oder mehrstückig ausgebildet sein. In diesem Beispiel ist er zweistückig ausgebildet, wobei der brustnahe Auflagebereich 82 und der dazu angrenzende erste Bereich 81 härter ausgebildet ist als der brustferne zweite Bereich 80. Der zweite Bereich 80 bildet den hinteren Gaumenteil, der erste Bereich 81 den vorderen Gaumenteil. Der hintere Gaumenteil 80 ist entsprechend nach unten gebogen ausgebildet und begrenzt bzw. verschliesst je nach Position die innere Kammer 5 zum ersten Vakuumanschluss 2 hin. Der vordere und der hintere Gaumenteil 81, 80 grenzen aneinander an und sind miteinander verbunden.

Im oberen Bereich zwischen Brusthaubenkörper 1 und den zwei Gaumenteilen 80, 81 ist ein Einstellelement 83 in Längsrichtung der Brusthaube verschiebbar gehalten. Mittels dieses Einstellelements 83 lässt sich die Härte des Gaumens variieren, indem seine Position bezüglich der zwei Gaumenteile 80, 81 verändert wird. In Figur 8a befindet es sich im brustfernen Bereich ausschliesslich über dem weicheren hinteren Gaumenteil 80 und hat einen Einfluss auf das Verhalten der Brusthaube während des Abpumpvorgangs. In den anderen Figuren ist das Einstellteil 83 näher zur Brust geschoben und überdeckt auch einen Teil des vorderen Gaumenteils 81. Der überdeckte Bereich wird somit versteift und in seiner Bewegung beschränkt. Das Verhalten des flexiblen Innenteils 8 während des Abpumpvorgangs wird beeinflusst. Die senkrecht nach unten gerichteten Pfeile in den Figuren 8a bis 8e zeigen die Position des Einstellelements 83.

In Figur 8f ist eine mögliche Ausführungsform für ein derartiges Einstellelement 83 dargestellt. Es ist ein Teilausschnitt eines steifen Hohlzylinders. Andere Formen sind möglich. Des Weiteren kann das Einstellteil 83 anstelle einer Verschiebung auch durch andere Bewegungsarten in die entsprechende Position gebracht werden. Anstelle eines mechanischen Einstellelements 83 lassen sich die Gaumenteile auch hohl ausbilden und ihre Steifheit durch Applikation eines Überdrucks variieren.

In Figur 8a ist die Brusthaube wiederum im Grundzustand bei Nichtgebrauch dargestellt. In Figur 8b wird die Brusthaube über die Brustwarze W gestülpt, so dass die Brustwarze W zwischen den zwei Gaumenteilen 80, 81 und dem Zungenteil 84 aufgenommen ist.

Gemäss Figur 8c wird anschliessend ein konstanter Unterdruck an die innere Kammer 5 angelegt und ein pulsierender Unterdruck unter dem Zugenteil 84, d.h. in der äusseren Kammer 6 appliziert.

Die Brustwarze W wird ähnlich wie in einem Mund eines Säuglings massiert und stimuliert, wobei auch hier die Längsausdehnung der Brustwarze W zum zweiten Vakuumanschluss 2 hin zwar erfolgt, aber begrenzt wird. Die Begrenzung erfolgt hier im Wesentlichen durch die nach unten gebogene Form des hinteren Gaumenteils 80. Die aus der Brustwarze W ausfliessende Milch ist in den Figuren gepunktet und mit einem Pfeil dargestellt. Diese Ausführungsform kann, wie auch die bereits beschriebenen, mit einem oder mehreren der oben beschriebenen Sensoren 7, 7' zur Detektion der Brustwarze W und des Verschlusses kombiniert werden.

In Figur 9 ist eine Variante der Druckapplikation dargestellt. Dabei ist eine Brusthaube vorhanden, bei welcher der Unterdruck in der äusseren Kammer 6 so angelegt wird, dass er zeitlich um 360° rotiert. In der Situation gemäss Figur 9 wirkt der Druck des flexiblen Innenteils 4 aktuell von unten auf die Brustwarze W. Dies ist durch den mit durchgezogenen Linien dargestellten geraden Pfeil veranschaulicht. Die gestrichelten Pfeile zeigen, dass der auf die Brustwarze W wirkende Druck um die Längsmittelachse L der Brusthaube rotiert und dass somit eine rotierende Massage und Stimulation der Brustwarze W stattfindet. Dies lässt sich durch entsprechende Ausbildung, z.B. durch Unterteilung der äusseren Kammer 6, einfach erreichen.

In den Figuren 10a bis 10e ist eine erfindungsgemässe Brustpumpeneinheit dargestellt. Sie weist, wie in Figur 10a gut erkennbar ist, einen steifen Grundkörper 1', eine flexible Brusthaube 4' sowie einen Milchsammelbehälter 9 auf. Die Brusthaube 4' und der Milchsammelbehälter 9 sind gemeinsam einstückig ausgebildet und bilden gemeinsam ein flexibles Element. Das flexible Element ist aus einem weichen Material, beispielsweise aus Silikon gefertigt. Die Wandstärke des flexiblen Elements ist relativ dünn, vorzugsweise ist es membran- oder folienähnlich.

Das flexible Element bildet einen Beutel mit einer Öffnung, deren Form geeignet ist, dichtend und ohne Falten zu bilden auf der Mutterbrust anzuliegen. Vorzugsweise ist die Öffnung rund, elliptisch oder oval. Der Rand der Öffnung ist vorzugsweise verstärkt, z.B. mit einer Einlageschnur oder durch verdickte Ausbildung. Dieser verstärkte Rand bildet den Auflagebereich 41 der Brusthaube 4', welcher bei Gebrauch dichtend an der Brust anliegt. Der vordere Teil, brustnahe Bereich des Beutels bildet somit die Brusthaube 4' mit dem flexiblen Grundkörper 40 und dem Auflagebereich 41. Der hintere, brustferne Bereich bildet den Milchsammelbehälter 9. Im mittleren Bereich des flexiblen Elements ist mindestens eine, vorzugsweise sind mehrere Taschen 49 ausgebildet. Ein steifer oder elastischer Ring 90 ist vorzugsweise vorhanden, welcher die brustfernen Enden der Taschen 49 umschliesst.

In Figur 10a ist der Grundkörper1' gut erkennbar. Er ist ringförmig gestaltet und weist eine zentrale Durchgangsöffnung 14 auf. Um diese zentrale Durchgangsöffnung 14 verteilt sind mehrere Federzungen 15 in Form von Blattfedern angeordnet, deren freie Enden zur zentralen Durchgangsöffnung 14 hin gerichtet sind und welche die zentrale Durchgangsöffnung 14 durchsetzen. Die Federzungen 15 sind mit Bolzen 18 am Grundkörper 1' befestigt. Im bolzennahen Bereich sind die Federzungen 15 mittels Spiralfedern 16 an der brustfernen Wandinnenseite des Grundkörpers 1' abgestützt. Dies ist in Figur 10e gut erkennbar. Auf jeder Federzunge 15 ist ferner eine Leine 17 oder Schnur befestigt, welche ebenfalls durch die zentrale Durchgangsöffnung 14 oder wie hier dargestellt, durch eine separate Öffnung 14' (siehe Figur 10d) durchgeführt ist. Es kann für jede Leine 17 eine eigene separate Öffnung 14' vorhanden sein. Diese Leinen 17 und separaten Öffnungen 14' sind in der Figur 10a nicht dargestellt.

Die Spiralfedern 16 können zwischen den Leinen 17 und den Bolzen 18 angeordnet sein, wie dies in den Figuren 10d bis 10f dargestellt ist. Es ist jedoch auch möglich, die Leinen 17 zwischen den Bolzen 18 und den Spiralfedern 16 anzuordnen, wie dies in Figur 10g der Fall ist.

Der Zusammenbau dieser erfindungsgemässen Brustpumpeneinheit sowie ihre Wirkungsweise lassen sich anhand der Figuren 10c bis 10f gut erläutern. In der Figur 10c ist das einstückige flexible Element dargestellt, welches die Brusthaube 4' mit dem Milchsammelbeutel 9 bildet. Es wird in der Praxis nicht so für sich alleine verwendet oder auf die Brust aufgesetzt. Die Figur erleichtert jedoch das Verständnis der Erfindung. Die Brusthaube umgibt vorzugsweise nicht nur die Brustwarze W sondern auch das umliegende Brustgewebe B. Es liegt vorzugsweise dichtend auf dem Brustgewebe B auf. Das flexible Element ist dabei so auf die Brustwarze W aufgelegt, dass sich die Taschen 49 im Bereich der Brustwarze befinden. Vorzugsweise überragen sie das Ende der Brustwarze W.

In Figur 10d ist nun die gesamte Brusthaubeneinheit erkennbar, wie sie in der Praxis auf die Brustwarze W aufgesetzt wird. Das flexible Element durchsetzt den Grundkörper 1', wobei die Federzungen 15 in die Taschen 49 des flexiblen Elements eingeschoben und darin gehalten sind. Die Leinen 17 sind verkürzt dargestellt. Sie enden üblicherweise gemeinsam in einer hier nicht dargestellten Zugvorrichtung, welche vorzugsweise manuell bedienbar ist. Die Zugvorrichtung ist beispielweise ein Knopf oder Stab, an welchen alle Leinen 17 befestigt sind und welcher in der Hand gehalten werden kann. Sie kann alternativ zum Bespiel in Form eines Schiebers, Teil eines hier ebenfalls nicht dargestellten Gehäuses ausgebildet sein.

In der Position gemäss Figur 10d wird die Brustwarze W von den Federzungen 15 umschlossen und leicht zusammengedrückt. Da sich die Federzungen 15 zum freien Ende der Brustwarze W hin zueinander neigen, ist die Längsdehnung der Brustwarze W begrenzt. In diesem Ausgangszustand vor dem Abpumpen der Milch ist der Milchsammelbehälter 9 zusammengedrückt. Es befindet sich keine Luft im Milchsammelbehälter 9. Der Bereich vor der Brustwarze W, gebildet durch die Federzungen 15, ist die innere Kammer 5.

In Figur 10e hat der Pumpvorgang begonnen. Durch Ziehen an den Leinen 17 lassen sich die Federzungen 15 entgegen der Kraft der Spiralfedern 16 anheben. Die Brustwarze W wird in ihrem Umfang freigegeben und kann sich ausdehnen und entspannen. Milch fliesst dadurch aus der Brustwarze W in die innere Kammer 5. Die Milch ist in den Figuren gepunktet dargestellt und mit dem Bezugszeichen M versehen. Durch Vermindern des Zugs auf die Leinen 17 werden die Federzungen 15 wieder abgesenkt und massieren die Brustwarze W. Dadurch wird zudem die extrahierte Milch M in den Milchsammelbehälter 9 gedrückt. Durch wiederholtes Anspannen und Loslassen der Leinen 17, d.h. durch wiederholtes Anheben und Absenken der Federzungen 15, wird die Brustwarze W massiert und stimuliert. Beim Loslassen erweitern sich die natürlichen Milchkanäle und Milch lässt sich optimal und ohne externe Saugquelle extrahieren.

Der Milchsammelbehälter 9 kann bereits als luftleerer Schlauch herstellerseitig geliefert werden. In Figur 10g ist eine Möglichkeit dargestellt, wie vor Gebrauch sichergestellt werden kann, dass der Milchsammelbehälter 9 luftleer ist. Der Grundkörper ist mit einem Deckel 11 versehen, welcher einerseits die Leinen 17 aufnimmt. Andererseits ist ein Presshebel 19 darin bewegbar. Der Presshebel 19 ist über ein Scharnier 190 mit einer Verlängerung 111 des Grundkörpers 1' verbunden. Die Verlängerung 111 sowie der Presshebel 19 bilden gemeinsam eine Aufnahme für den Milchsammelbehälter 9. Wird nun der Presshebel 19 in Richtung Verlängerung 111 gedrückt, so wird der Milchsammelbehälter 9 zusammengepresst und allfällige verbleibende Restluft wird über die Brusthaube 4' ausgepresst. Während der Extraktion der Muttermilch wird anschliessend der Presshebel 19 wieder freigegeben.

In Figur 11 ist eine weitere Ausführungsform der erfindungsgemässen Brusthaube dargestellt. Es lässt sich hierfür eine der oben beschriebenen Brusthaube oder eine Brusthaube bekannter Art verwenden. Der Brusthaubenkörper 1 ist hier lediglich schematisch dargestellt. Er kann eine andere Form und Grösse aufweisen. Insbesondere kann er wie die bekannten klassischen Brusthauben, auch einen grösseren Bereich des Brustgewebes aufnehmen.

Erfindungsgemäss ist die Brusthaube mit Luftaustrittsöffnungen 110 versehen, durch welche aktiv Luft in Richtung Brust strömt. D.h. die Brust wird durch die Brusthaube angeblasen. Als entsprechenden Lüfter lässt sich beispielweise ein Auspuff des Brustpumpenaggregats verwenden oder der Lüfter kann ein Gebläse oder Ventilator sein, welches bzw. welcher am oder im Brusthaubenkörper angeordnet ist. Andere Arten zur Ausbildung des Lüfters zwecks Erzeugung eines Luftstroms sind möglich.

In Figur 11 ist rein schematisch ein Aufsatz 100 auf den Brusthaubenkörper 1 aufgesetzt, wobei der Aufsatz 100 die Luftaustrittsöffnungen 110 zur Luftbeaufschlagung der Mutterbrust aufweist. Vorzugsweise befinden sich die Luftaustrittsöffnungen nur in einem Teilbereich des Umfangs der Brusthaube, um eine Nase des Säuglings zu simulieren.

In den Figuren 12a und 12b ist eine weitere Ausführungsform der erfindungsgemässen Brusthaube in zwei Varianten dargestellt. Sie weist wiederum einen Brusthaubenkörper 1 und ein flexibles Innenteil 4 mit einem Auflagebereich 41 auf. Die Brusthaube umgibt wiederum maximal die Brustwarze W und die Areola. Die Brustwarze W ist eng vom Grundkörper 40 des flexiblen Innenteils 4 umfasst.

In der Ausführungsform gemäss Figur 12a wird über den ersten Vakuumanschluss 2 ein zeitlich konstantes oder annähernd konstantes Vakuum in der inneren Kammer 5, in welche die Milch fliesst, angelegt. Über den zweiten Vakuumanschluss 3 wird ein pulsierendes Vakuum in der äusseren Kammer 6, welche die Brustwarze W umgibt, angelegt. Die Brustwarze W wird massiert und die natürlichen Milchkanäle öffnen und schliessen sich während des Abpumpvorgangs.

In der Ausführungsform gemäss Figur 12b wird über den ersten Vakuumanschluss 2 ein pulsierendes Vakuum und über den zweiten Vakuumanschluss 3 ein zeitlich konstantes oder annähernd konstantes Vakuum angelegt. Auf diese Weise werden die natürlichen Milchkanäle während des gesamten Abpumpvorgangs offen gehalten, da die Brustwarze W aufgrund des Unterdrucks in der äusseren Kammer 6 radial nach aussen gezogen wird. Der Grundkörper 40 des Innenteils 4 in der Ausführungsform gemäss Figur 12b weist eine oder mehrere Unterbrechungen 40' auf.

In beiden Ausführungsformen gemäss den Figuren 12a und 12b wird die Brustwarze W vom Grundkörper 40 des flexiblen Innenteils ringförmig umschlossen, welcher wie ein Mund eines Säuglings die Brustwarze W fest umschliesst.

In Figur 13 ist schematisch eine Brustpumpeneinheit dargestellt, wie sie mit Ausnahme der Ausführungsform gemäss den Figuren 10a bis 10g mit den oben genannten Brusthauben verwendet werden kann. Sie weist die Brusthaube auf, hier mit dem Brusthaubenkörper 1. Der erste Vakuumanschluss 2 des Brusthaubenkörpers 1 ist mit einer ersten Vakuumleitung 21 mit einer Vakuumpumpe 200 verbunden. Die Vakuumpumpe 200 kann eine oder mehrere Pumpaggregate sowie eine Steuerungseinheit 201 aufweisen. Sind Sensoren in der Brusthaube vorhanden, so empfängt die Steuerungseinheit die Daten der Sensoren und steuert das mindestens eine Pumpaggregat entsprechend.

Der zweite Vakuumanschluss 3 ist über eine zweite Vakuumleitung 31 ebenfalls mit der Vakuumpumpe 200 verbunden. Eine Milchleitung 91 führt von der Vakuumpumpe zum Milchsammelbehälter 9. Wie oben dargelegt, kann bei allen beschriebenen Brusthauben, mit Ausnahme der Ausführungsform gemäss den Figuren 10a bis 10g, entweder ein separater Milchanschluss aus der inneren Kammer 5 direkt oder über eine Leitung zu einem Milchsammelbehälter führen. Es ist jedoch auch möglich, wie hier dargestellt, den ersten Vakuumanschluss 2 als Milchanschluss zu benützen und die Milch durch die erste Vakuumleitung zur Brustpumpe oder einer vorgelagerten Kammer und von dort direkt oder über eine Milchleitung 91 in den Milchsammelbehälter 9 zu führen. Alternative Wege für die extrahierte Milch sind ferner möglich.

Die erfindungsgemässen Brustpumpeneinheiten sowie die erfindungsgemässen Brusthauben ermöglichen eine maximierte Abpumpleistung sowie eine minimierte Abpumpdauer pro Pumpsitzung.

**BEZUGSZEICHENLISTE**

| | | | |
|---|---|---|---|
| 1 | Brusthaubenkörper | 41 | Auflagebereich |
| 1' | Grundkörper | 410 | Hohlraum |
| 10 | Basis | 42 | Befestigungsflansch |
| 100 | Aufsatz | 43 | Rückhalteelement |
| 110 | Luftaustrittsöffnungen | 44 | Verschluss |
| 111 | Verlängerung | 440 | Bogen |
| 11 | Deckel | 45 | Kontaktierungsbereich |
| 12 | Anschlussstutzen | 46 | Tasche |
| 14 | Durchgangsöffnung | 47 | Trennwand |
| 14' | separate Öffnung | 49 | Aufnahmetasche |
| 15 | Federzunge | | |
| 16 | Spiralfeder | 5 | innere Kammer |
| 17 | Leine | | |
| 18 | Bolzen | 6 | äussere Kammer |
| 19 | Presshebel | | |
| 190 | Scharnier | 7 | erster Sensor |
| | | 7' | zweiter Sensor |
| 2 | erster Vakuumanschluss | | |
| 21 | erste Vakuumleitung | 8 | flexibles Innenteil |
| 200 | Vakuumpumpe | 80 | hinteres Gaumenteil |
| 201 | Steuerungseinheit | 81 82 | vorderes Gaumenteil Auflagebereich |
| 3 | zweiter Vakuumanschluss | 83 | Einstellelement |
| 30 | dritter Vakuumanschluss | 84 | Zungenteil |
| 31 | zweite Vakuumleitung | | |
| | | 9 | Milchsammelbehälter |
| 4 | flexibles Innenteil | 90 | Ring |
| 4' | Brusthaube | 91 | Milchleitung |
| 40 | Grundkörper | | |
| 40' | Unterbrechung | B | Brust |
| 400 | Bogen | M | Milch |
| W | Brustwarze | W_{G} | grosse Brustwarze |
| W_{N} | durchschnittliche Brustwarze | L | Längsmittelachse |
| W_{K} | kleine Brustwarze | | |

## Patentansprüche

1. Brusthaube einer Brustpumpeneinheit zum Abpumpen von menschlicher Muttermilch, wobei die Brustpumpeneinheit eine Vakuumpumpe zur Erzeugung von Drücken aufweist, wobei die Brusthaube einen Auflagebereich (41) zur dichtenden Auflage auf die menschliche Mutterbrust und eine innere Kammer (5) zur Aufnahme einer Brustwarze (W) der Mutterbrust aufweist, **dadurch gekennzeichnet, dass** die Brusthaube mit mindestens einem Sensor (7, 7) versehen ist zur Bestimmung der Position der Brustwarze (W) und/oder der Position eines Verschlusses der inneren Kammer (5) während des Abpumpvorgangs.

2. Brusthaube nach Anspruch 1, wobei die innere Kammer (5) eine Längsachse (L) aufweist, wobei die innere Kammer (5) nach Massgabe eines angelegten Drucks kollabiert, wobei mindestens einer des mindestens einen Sensors (7, 7') zur Bestimmung der Lage bezüglich der Längsachse (L), an welcher die innere Kammer (5) kollabiert, ausgebildet ist.

3. Brusthaube nach Anspruch 1, wobei der Auflagebereich (41) brustseitig in einem umlaufenden, weichen und dichtenden Kissen endet.

4. Brusthaube nach Anspruch 3, wobei das Kissen einen umlaufenden aufblasbaren Hohlraum (410) aufweist.

5. Brusthaube nach Anspruch 1, wobei die Brusthaube brustseitig eine Aufnahmetasche (46) zum Auffangen von Muttermilchtropfen beim Entfernen der Brusthaube aufweist.

6. Brusthaube nach Anspruch 5, wobei die Brusthaube ein flexibles Innenteil (4) aufweist, welches den Auflagebereich (41) zur dichtenden Auflage auf die menschliche Mutterbrust und eine innere Kammer (5) zur Aufnahme einer Brustwarze (W) der Mutterbrust bildet, wobei die Tasche (46) in dem flexiblen Innenteil (4) ausgebildet ist.

7. Brusthaube nach Anspruch 1, wobei die Brusthaube einen äusseren Brusthaubenkörper (1) und ein flexibles Innenteil (4) aufweist, wobei das flexible Innenteil (4)
- den Auflagebereich (41) zur dichtenden Auflage auf die menschliche Mutterbrust bildet und
- die Brusthaube in die innere Kammer (5) und in mindestens eine äussere Kammer (6), welche die Brustwarze (W) mindestens teilweise umgibt, unterteilt,
wobei die innere Kammer (5) zur Aufnahme eines ersten Drucks der Vakuumpumpe (200) ausgebildet ist und die mindestens eine äussere Kammer (6) zur Aufnahme mindestens eines zweiten Drucks der Vakuumpumpe (200) ausgebildet ist, wobei das flexible Innenteil (4) einstückig ausgebildet ist und die Brusthaube eine weitere Kammer in Form eines Hohlraums (410) aufweist, welche von der mindestens einen äusseren Kammer (6) unterteilt ist, indem eine feste oder lösbare Verbindung zwischen dem flexiblen Innenteil (4) und dem Brusthaubenkörper (1) eine umlaufende Trennwand (47) bildet, und wobei die zusätzliche Kammer (410) im Auflagebereich (41) der Brusthaube angeordnet ist.

8. Brusthaube nach Anspruch 1, wobei die Brusthaube eine Längsachse (L) definiert, wobei die innere Kammer (5) durch einen äusseren Bereich (80, 81, 82, 84) begrenzt ist, welcher asymmetrisch ausgebildet ist, und wobei mindestens ein Teilbereich (84) des äusseren Bereichs eine äussere Kammer (6) aufweist, deren Innenseite mit einem Druck beaufschlagbar ist.

9. Brusthaube nach Anspruch 1, wobei die Brusthaube ferner einen Lüfter aufweist, welcher Luft in Richtung Mutterbrust bläst.

10. Brusthaube nach Anspruch 1, wobei die Brusthaube mindestens eine äussere Kammer (6) aufweist, welche die Brustwarze (W) mindestens teilweise umgibt, wobei die innere Kammer (5) zur Aufnahme eines ersten Drucks der Vakuumpumpe ausgebildet ist und die mindestens eine äussere Kammer (6) zur Aufnahme mindestens eines zweiten Drucks der Vakuumpumpe ausgebildet ist, wobei der erste Druck ein annähernd zeitlich konstanter Druck und der mindestens eine zweite Druck ein pulsierender Druck ist.

11. Brusthaube nach Anspruch 10, wobei die Brusthaube ein flexibles Innenteil (4) aufweist, welches die Brusthaube in die innere Kammer (5) und die mindestens eine äussere Kammer (6) unterteilt und wobei das flexible Innenteil (4) von innen mit dem ersten Druck und von aussen mit dem mindestens einen zweiten Druck beaufschlagbar ist.

12. Brusthaube nach Anspruch 11, wobei das flexible Innenteil (4) eine flexible Einlage ist, welche fest oder lösbar mit einem steifen oder halbsteifen Brusthaubenkörper (1) verbunden ist.

13. Brusthaube nach Anspruch 1, wobei die innere Kammer (5) über einen gesamten Aufnahmebereich konisch ausgebildet ist.

14. Brusthaube nach Anspruch 13, wobei die innere Kammer (5) eine innere Wand (40) aufweist, welche mit Rückhaltemitteln (43) zum Rückhalten der Brustwarze (W) während des Pumpvorgangs ausgestattet ist.

15. Brustpumpeneinheit zum Abpumpen von menschlicher Muttermilch wobei die Brustpumpeneinheit eine Vakuumpumpe (200) mit mindestens einem Pumpaggregat zur Erzeugung von Drücken und mindestens eine Brusthaube gemäss einem der Ansprüche 1 bis 14 aufweist, wobei die Brusthaube eine innere Kammer (5) zur Aufnahme einer Brustwarze (W) der Mutterbrust sowie mindestens eine äussere Kammer (6) aufweist, welche die Brustwarze (W) mindestens teilweise umgibt, wobei die innere Kammer (5) zur Aufnahme eines ersten Drucks der Vakuumpumpe (200) ausgebildet ist und die mindestens eine äussere Kammer (6) zur Aufnahme mindestens eines zweiten Drucks der Vakuumpumpe (200) ausgebildet ist, wobei die Vakuumpumpe (200) ausgebildet ist, um den ersten Druck in Form eines annähernd zeitlich konstanten Drucks und den mindestens einen zweiten Druck in Form eines pulsierenden Drucks zu erzeugen.

16. Brustpumpeneinheit nach Anspruch 15, wobei mindestens ein Sensor (7, 7') vorhanden ist zur Bestimmung der Position der Brustwarze (W) während des Abpumpvorgangs und wobei eine Steuerung (201) vorhanden ist, welche ausgebildet ist, den ersten Druck und/oder den mindestens einen zweiten Druck nach Massgabe dieser bestimmten Position der Brustwarze (W) zu variieren.

17. Brusthaubeneinheit einer Brustpumpeneinheit zum Abpumpen von menschlicher Muttermilch, wobei die Brustpumpeneinheit eine Vakuumpumpe (200) zur Erzeugung von Drücken aufweist, wobei die Brusthaubeneinheit eine Brusthaube gemäss einem der Ansprüche 1 bis 14 umfasst, wobei die innere Kammer (5) eine erste Öffnung zur Aufnahme der Brustwarze (W) und als ausschliessliche weitere Öffnung eine Verbindungsöffnung zu einem Milchsammelbehälter (9) aufweist, wobei die Brusthaube über diese Öffnung luftdicht mit dem Milchsammelbehälter (9) verbunden ist, **dadurch gekennzeichnet, dass** Mittel (15, 17) vorhanden sind, welche die innere Kammer (5) zyklisch vergrössern zwecks Erzeugung eines Unterdrucks in der inneren Kammer (5) zum Abpumpen der Muttermilch.

18. Brusthaubeneinheit nach Anspruch 17, wobei die Mittel Federzungen (15) und die Federzungen (15) betätigende Leinen (17) sind.

## Claims

1. Breast shield of a breastpump unit for expression of human breastmilk, wherein the breastpump unit has a vacuum pump for generating pressures, wherein the breast shield has an application region (41) for sealing application to the human mother's breast and an inner chamber (5) for receiving a nipple (W) of the mother's breast, **characterized in that** the breast shield is provided with at least one sensor (7, 7') for determining the position of the nipple (W) and/or the position of a closure of the inner chamber (5) during the pumping operation.

2. Breast shield as claimed in claim 1, wherein the inner chamber (5) has a longitudinal axis (L) and wherein the inner chamber (5) collapses in accordance with an applied pressure, wherein the breast shield is provided with at least one of the sensors (7, 7') is configured to determine the position with regard to the longitudinal axis (L) at which the inner chamber (5) collapses.

3. Breast shield as claimed in claim 1, wherein the application region (41) ends in an encircling, soft and sealing pad on the breast side.

4. Breast shield as claimed in claim 3, wherein the pad has an encircling inflatable cavity (410).

5. Breast shield as claimed in claim 1, wherein the breast shield has a receiving pocket (46) on the breast side for collecting breastmilk drops when the breast shield is removed.

6. Breast shield as claimed in claim 5, wherein the breast shield has a flexible inner part (4) which forms the application region (41) for sealing application to the human mother's breast and an inner chamber (5) for receiving a nipple (W) of the mother's breast, wherein the pocket (46) is formed in the flexible inner part (4).

7. Breast shield as claimed in claim 1, wherein the breast shield has an outer breast shield body (1) and a flexible inner part (4), wherein the flexible inner part (4)
- forms an application region (41) for sealing application to the human mother's breast, and
- subdivides the breast shield into the inner chamber (5) and into at least one outer chamber (6) which at least partially surrounds the nipple (W),
wherein the inner chamber (5) is configured to be subjected to a first pressure of the vacuum pump (200) and the at least one outer chamber (6) is configured to be subjected to at least one second pressure of the vacuum pump (200), wherein the flexible inner part (4) is configured in one piece and the breast shield has a further chamber in the form of a cavity (410) which is subdivided by the at least one outer chamber (6), wherein a fixed or releasable connection between the flexible inner part (4) and the breast shield body (1) forms an encircling partition wall (47), and wherein the additional chamber (410) is arranged in the application region (41) of the breast shield.

8. Breast shield as claimed in claim 1, wherein the inner chamber (5) is bounded by an outer region (80, 81, 82, 84) which is formed in an asymmetrical manner, and wherein at least one subregion (84) of the outer region has an outer chamber (6), the inner side of which is able to be subjected to a pressure.

9. Breast shield as claimed in claim 1, wherein the breast shield also has a fan which blows air in the direction of the breast.

10. Breast shield as claimed in claim 1, wherein the breast shield has at least one outer chamber (6) which at least partially surrounds the nipple (W), wherein the inner chamber (5) is configured to be subjected to a first pressure of the vacuum pump and the at least one outer chamber (6) is configured to be subjected to at least one second pressure of the vacuum pump, wherein the first pressure is an approximately temporally constant pressure and the at least one second pressure is a pulsating pressure.

11. Breast shield as claimed in claim 10, wherein the breast shield has a flexible inner part (4) which subdivides the breast shield into the inner chamber (5) and the at least one outer chamber (6), and wherein the flexible inner part (4) is able to be subjected to the first pressure from the inside and to the at least one second pressure from the outside.

12. Breast shield as claimed in claim 11, wherein the flexible inner part (4) is a flexible insert which is connected fixedly or releasably to a rigid or semirigid breast shield body (1).

13. Breast shield as claimed in claim 1, wherein the inner chamber (5) is configured in a conical manner over an entire receiving region.

14. Breast shield as claimed in claim 13, wherein the inner chamber (5) has an inner wall (40) which is equipped with retaining means (43) for retaining the nipple (W) during the pumping operation.

15. Breastpump unit for expression of human breastmilk, wherein the breastpump unit has a vacuum pump (200) with at least one pump aggregate for generating pressures and at least one breast shield as claimed in one of claims 1 to 14, wherein the breast shield has an inner chamber (5) for receiving a nipple (W) of the breast and also at least one outer chamber (6) which at least partially surrounds the nipple (W), wherein the inner chamber (5) is configured to be subjected to a first pressure of the vacuum pump (200) and the at least one outer chamber (6) is configured to be subjected to at least one second pressure of the vacuum pump (200), wherein the vacuum pump is configured to generate the first pressure in the shape of a approximately temporally constant pressure and to generate the second pressure in the shape of a pulsating pressure.

16. Breastpump unit as claimed in claim 15, wherein at least one sensor (7, 7') is provided to determine the position of the nipple (W) during the pumping operation, and wherein a controller (201) is provided which is configured to vary the first pressure and/or the at least one second pressure in accordance with this determined position of the nipple (W).

17. Breast shield unit of a breastpump unit for expression of human breastmilk, wherein the breastpump unit has a vacuum pump (200) for generating pressures, , wherein the breast shield unit comprises a breast shield as claimed in one of claims 1 to 14, wherein the inner chamber (5) has a first opening for receiving the nipple (W) and, as the only other opening, a connecting opening to a milk collection container (9), wherein the breast shield is connected to the milk collection container (9) in an airtight manner via this opening, **characterized in that** means (15, 17) are provided which cyclically enlarge the inner chamber (5) for the purpose of generating a negative pressure in the inner chamber (5) for expression of the breastmilk.

18. Breast vshield unit as claimed in claim 17, wherein the means are flexible tongues (15) and lines (17) that actuate the flexible tongues (15).

## Revendications

1. Téterelle d'une unité tire-lait destinée à tirer du lait maternel humain, l'unité tire-lait présentant une pompe à vide pour générer des pressions, la téterelle présentant une zone d'application (41) destinée à une application étanche contre le sein humain et une chambre intérieure (5) destinée à recevoir un mamelon (W) du sein, **caractérisée en ce que** la téterelle est munie d'au moins un capteur (7, 7) servant à déterminer la position du mamelon (W) et/ou la position d'une fermeture de la chambre intérieure (5) pendant l'expression.

2. Téterelle selon la revendication 1, dans laquelle la chambre intérieure (5) présente un axe longitudinal (L), la chambre intérieure (5) s'affaissant en fonction d'une pression exercée, au moins l'un du au moins un capteur (7, 7') étant réalisé pour déterminer la position par rapport à l'axe longitudinal (L) à laquelle la chambre intérieure (5) s'affaisse.

3. Téterelle selon la revendication 1, dans laquelle la zone d'application (41) se termine côté poitrine par un bourrelet périphérique, doux et étanche.

4. Téterelle selon la revendication 3, dans laquelle le bourrelet présente une cavité périphérique gonflable (410).

5. Téterelle selon la revendication 1, dans laquelle la téterelle présente côté poitrine une poche de réception (46) destinée à recueillir des gouttes de lait maternel lors du retrait de la téterelle.

6. Téterelle selon la revendication 5, dans laquelle la téterelle présente une pièce intérieure flexible (4) qui forme la zone d'application (41) pour l'application étanche contre le sein humain et une chambre intérieure (5) pour recevoir un mamelon (W) du sein, la poche (46) étant réalisée dans la pièce intérieure flexible (4).

7. Téterelle selon la revendication 1, dans laquelle la téterelle présente un corps de téterelle extérieur (1) et une pièce intérieure flexible (4), dans laquelle la pièce intérieure flexible (4)
- forme la zone d'application (41) pour l'application étanche contre le sein humain, et
- divise la téterelle en la chambre intérieure (5) et au moins une chambre extérieure (6) qui entoure le mamelon (W) au moins en partie,
dans laquelle la chambre intérieure (5) est réalisée pour recevoir une première pression de la pompe à vide (200), et ladite au moins une chambre extérieure (6) est réalisée pour recevoir au moins une deuxième pression de la pompe à vide (200), la pièce intérieure flexible (4) étant réalisée d'un seul tenant, et la téterelle présentant une autre chambre sous la forme d'une cavité (410) qui est divisée par ladite au moins une chambre extérieure (6) en ce qu'une liaison fixe ou amovible forme une cloison périphérique (47) entre la pièce intérieure flexible (4) et le corps de téterelle (1), et la chambre supplémentaire (410) étant disposée dans la zone d'application (41) de la téterelle.

8. Téterelle selon la revendication 1, dans laquelle la téterelle définit un axe longitudinal (L), la chambre intérieure (5) étant limitée par une zone extérieure (80, 81, 82, 84) qui est réalisée de manière asymétrique, et dans laquelle au moins une zone partielle (84) de la zone extérieure présente une chambre extérieure (6) dont le côté intérieur peut être sollicité par une pression.

9. Téterelle selon la revendication 1, dans laquelle la téterelle présente en outre un ventilateur qui souffle de l'air en direction du sein.

10. Téterelle selon la revendication 1, dans laquelle la téterelle présente au moins une chambre extérieure (6) qui entoure le mamelon (W) au moins en partie, la chambre intérieure (5) étant réalisée pour recevoir une première pression de la pompe à vide, et ladite au moins une chambre extérieure (6) étant réalisée pour recevoir au moins une deuxième pression de la pompe à vide, la première pression étant une pression approximativement constante dans le temps et ladite au moins une deuxième pression étant une pression pulsatoire.

11. Téterelle selon la revendication 10, dans laquelle la téterelle présente une pièce intérieure flexible (4) qui divise la téterelle en la chambre intérieure (5) et ladite au moins une chambre extérieure (6), et dans laquelle la pièce intérieure flexible (4) peut être sollicitée de l'intérieur par la première pression et de l'extérieur par ladite au moins une deuxième pression.

12. Téterelle selon la revendication 11, dans laquelle la pièce intérieure flexible (4) est un insert flexible qui est relié de manière fixe ou amovible à un corps de téterelle (1) rigide ou semi-rigide.

13. Téterelle selon la revendication 1, dans laquelle la chambre intérieure (5) est réalisée de manière conique sur toute une zone de réception.

14. Téterelle selon la revendication 13, dans laquelle la chambre intérieure (5) présente une paroi intérieure (40) qui est équipée de moyens de retenue (43) pour retenir le mamelon (W) pendant l'expression.

15. Unité tire-lait destinée à tirer du lait maternel humain, l'unité tire-lait présentant une pompe à vide (200) dotée d'au moins un groupe de pompage destiné à générer des pressions et au moins une téterelle selon l'une quelconque des revendications 1 à 14, la téterelle présentant une chambre intérieure (5) destinée à recevoir un mamelon (W) du sein ainsi qu'au moins une chambre extérieure (6) qui entoure le mamelon (W) au moins en partie, la chambre intérieure (5) étant réalisée pour recevoir une première pression de la pompe à vide (200) et ladite au moins une chambre extérieure (6) étant réalisée pour recevoir au moins une deuxième pression de la pompe à vide (200), la pompe à vide (200) étant réalisée pour générer la première pression sous la forme d'une pression approximativement constante dans le temps et pour générer ladite au moins une deuxième pression sous la forme d'une pression pulsatoire.

16. Unité tire-lait selon la revendication 15, dans laquelle au moins un capteur (7, 7') est présent pour déterminer la position du mamelon (W) pendant l'expression, et dans laquelle une commande (201) est présente qui est réalisée pour faire varier la première pression et/ou ladite au moins une deuxième pression en fonction de cette position déterminée du mamelon (W).

17. Unité de téterelle d'une unité tire-lait destinée à tirer du lait maternel humain, l'unité tire-lait présentant une pompe à vide (200) destinée à générer des pressions, l'unité de téterelle comprenant une téterelle selon l'une quelconque des revendications 1 à 14, la chambre intérieure (5) présentant une première ouverture destinée à recevoir le mamelon (W) et comme autre ouverture unique une ouverture de liaison avec un récipient de collecte de lait (9), la téterelle étant reliée de manière étanche au récipient de collecte de lait (9) par l'intermédiaire de cette ouverture, **caractérisée en ce que** des moyens (15, 17) sont présents qui agrandissent de manière cyclique la chambre intérieure (5) en vue de générer une dépression dans la chambre intérieure (5) pour tirer le lait maternel.

18. Unité tire-lait selon la revendication 17, dans laquelle les moyens sont des languettes élastiques (15) et des cordelettes (17) actionnant les languettes élastiques (15).
